# EUROPEAN PATENT SPECIFICATION

(11) **EP 0 569 193 B1**
(45) Date of publication and mention of the grant of the patent: **26.02.1997**
(21) Application number: 93303373.0
(22) Date of filing: 29.04.1993
(51) Int. Cl.: C07D 261/16, A61K 31/42

(54) **N-isoxazole-phenylsulfonamide derivatives and their use as endothelin antagonists**
N-isoxazol-phenylsulfonamid Derivate und ihre Anwendung als Endothelin-Antagonisten
Dérivés de N-isoxazole-phénylsulfonamide et leur utilisation comme antagonistes de l'endothélin

(30) Priority: 06.05.1992 US 879000
(43) Date of publication of application: 10.11.1993
(73) Proprietor: E.R. SQUIBB & SONS, INC., Princeton New Jersey 08543-4000 (US)
(72) Inventor: Murugesan, Natesan, Lawrenceville, New Jersey (US); Hunt, John T., Princeton, New Jersey (US)
(74) Representative: Thomas, Roger Tamlyn

(56) References cited:
- EP-A- 510 526
- EP-A- 526 708
- EP-A- 558 258
- CHEMICAL ABSTRACTS, vol. 70, no. 19, 12 May 1969, Columbus, Ohio, US; abstract no. 87639g, SAITO NORIO ET AL 'Synthesis of fluorine-containing sulfonamide derivatives' page 324; & YAKUGAKU ZASSHI vol. 88, no.12, 1968, pages 1610-1615
- CHEMICAL ABSTRACTS, vol. 73, no. 23, 7 December 1970, Columbus, Ohio, US; abstract no. 120511w, page 368; & JP-B-7 023 726

## Description

### Field of the Invention

This invention relates to endothelin antagonists useful, inter alia, for treatment of hypertension.

### Brief Description of the Invention

Compounds of the formula 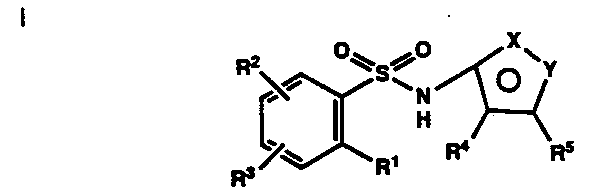 and pharmaceutically acceptable salts thereof are endothelin receptor antagonists useful, inter alia, as antihypertensive agents. Throughout this specification, the above symbols are defined as follows:
one of X and Y is N and the other is O;
R¹, R² and R³ are each independently
   (a) hydrogen, except that R¹ is other than hydrogen;
   (b) alkyl, alkenyl, alkynyl, alkoxy, cycloalkyl, cycloalkylalkyl, cycloalkenyl, cycloalkenylalkyl, aryl, aryloxy, aralkyl, or aralkoxy, any of which may be substituted with Z¹, Z² and Z³;
   (c) halo;
   (d) hydroxyl;
   (e) cyano;
   (f) nitro;
   (g) -C(O)H or -C(O)R⁶;
   (h) -CO₂H or -CO₂R⁶;
   (i) -SH, -S(O)ₙR⁶, -S(O)ₘ-OH, -S(O)ₘ-OR⁶, -O-S(O)ₘ-R⁶, -O-S(O)ₘOH, or -O-S(O)ₘ-OR⁶;
   (j) -Z⁴-NR⁷R⁸; or
   (k) -Z⁴-N(R¹¹)-Z⁵-NR⁹R¹⁰;
R⁴ and R⁵ are each independently
   (a) hydrogen;
   (b) alkyl, alkenyl, alkynyl, alkoxy, cycloalkyl, cycloalkylalkyl, cycloalkenyl, cycloalkenylalkyl, aryl, aryloxy, aralkyl, or aralkoxy, any of which may be substituted with Z¹, Z² and Z³;
   (c) halo;
   (d) hydroxyl;
   (e) cyano;
   (f) nitro;
   (g) -C(O)H or -C(O)R⁶;
   (h) -CO₂H or -CO₂R⁶;
   (i) -SH, -S(O)ₙR⁶, -S(O)ₘ-OH, -S(O)ₘ-OR⁶, -O-S(O)ₘ-R⁶, -O-S(O)ₘOH, or -O-S(O)ₘ-OR⁶;
   (j) -Z⁴-NR⁷R⁸;
   (k) -Z⁴-N(R¹¹)-Z⁵-NR⁹R¹⁰; or
   (l) R⁴ and R⁵ together are alkylene or alkenylene (either of which may be substituted with Z¹, Z² and Z³), completing a 4- to 8-membered saturated, unsaturated or aromatic ring together with the carbon atoms to which they are attached;
R⁶ is alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
R⁷ is
   (a) hydrogen;
   (b) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
   (c) cyano;
   (d) hydroxyl;
   (e) -C(O)H or -C(O)R⁶;
   (f) -CO₂R⁶;
   (g) -SH, -S(O)nR⁶, -S(O)m-OH, -S(O)m-OR⁶, -O-S(O)m-R⁶, -O-S(O)mOH, or -O-S(O)m-OR⁶, except when Z⁴ is -S(O)ₙ-;
R⁸ is
   (a) hydrogen;
   (b) -C(O)H or -C(O)R⁶, except when Z⁴ is -C(O)- and R⁷ is -C(O)H, -C(O)R⁶, or -CO₂R⁶;
   (c) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³; or
R⁷ and R⁸ together are alkylene or alkenylene (either of which may be substituted with Z¹, Z² and Z³), completing a 3- to 8-membered saturated, unsaturated or aromatic ring together with the nitrogen atom to which they are attached;
R⁹ is
   (a) hydrogen;
   (b) hydroxyl;
   (c) -C(O)H or -C(O)R⁶;
   (d) -CO₂R⁶;
   (e) -SH, -S(O)ₙR⁶, -S(O)ₘ-OH, -S(O)ₘ-OR⁶, -O-S(O)ₘ-R⁶, -O-S(O)ₘOH, or -O-S(O)ₘ-OR⁶;
   (f) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
R¹⁰ is
   (a) hydrogen;
   (b) -C(O)H or -C(O)R⁶, except when Z⁵ is -C(O)- and R⁹ is -C(O)H, -C(O)R⁶, or -CO₂R⁶; or
   (c) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
R¹¹ is
   (a) hydrogen;
   (b) hydroxyl;
   (c) -C(O)H, -C(O)R⁶ or CO₂R⁶; or
   (d) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
   or any two of R⁹, R¹⁰ and R¹¹ together are alkylene or alkenylene (either of which may be substituted with Z¹, Z² and Z³),
   completing a 3- to 8-membered saturated, unsaturated or aromatic ring together with the atoms to which they are attached;
Z¹, Z² and Z³ are each independently
   (a) hydrogen;
   (b) halo;
   (c) hydroxy;
   (d) alkyl;
   (e) alkenyl;
   (f) aralkyl;
   (g) alkoxy;
   (h) aryloxy;
   (i) aralkoxy;
   (j) -SH, -S(O)ₙZ⁶, -S(O)m-OH, -S(O)ₘ-OZ⁶, -O-S(O)ₘ-Z⁶, -O-S(O)ₘOH, or -O-S(O)ₘ-OZ⁶;
   (k) oxo;
   (l) nitro;
   (m) cyano;
   (n) -C(O)H or -C(O)Z⁶;
   (o) -CO₂H or -CO₂Z⁶;
   (p) -Z⁴-NZ⁷Z⁸;
   (q) -Z⁴-N(Z¹¹),Z⁵,Z⁶; or
   (r) -Z⁴-N(Z¹¹)-Z⁵-NZ⁷Z⁸;
Z⁴ and Z⁵ are each independently
   (a) a single bond;
   (b) -Z⁹-S(O)ₙ-Z¹⁰-;
   (c) -Z⁹-C(O)-Z¹⁰-;
   (d) -Z⁹-C(S)-Z¹⁰-;
   (e) -Z⁹-O-Z¹⁰-;
   (f) -Z⁹-S-Z¹⁰-; or
   (g) -Z⁹-O-C(O)-Z¹⁰-;
Z⁶, Z⁷ and Z⁸ are each independently hydrogen, alkyl, alkenyl, alkynyl, cycloalkyl, cycloalkylalkyl, cycloalkenyl, cycloalkenylalkyl, aryl, or aralkyl, or Z⁷ and Z⁸ together are alkylene or alkenylene, completing a 3- to 8-membered saturated, unsaturated or aromatic ring together with the nitrogen atom to which they are attached;
Z⁹ and Z¹⁰ are each independently a single bond, alkylene, alkenylene, or alkynylene;
Z¹¹ is
   (a) hydrogen;
   (b) hydroxyl;
   (c) -C(O)H, -C(O)Z⁶ or -CO₂Z⁶;
   (d) alkyl, alkenyl, alkynyl, alkoxy, cycloalkyl, cycloalkylalkyl, cycloalkenyl, cycloalkenylalkyl, aryl, or aralkyl;
   or any two of Z⁷, Z⁸ and Z¹¹ together are alkylene or alkenylene, completing a 3- to 8-membered saturated, unsaturated, or aromatic ring together with the atoms to which they are attached;
m is 1 or 2; and
n is 0, 1, or 2.

The invention as defined in the US application from which priority is claimed is set out in Claim 11.

Yakugaku Zasshi (1968) 88, 1610-1615 and Chemical Abstracts (1970) 73, 120511w disclose 4-amino-2-fluoro-N-(5-methyl-3-isoxazolyl)benzenesulfonamide, 2-amino-N-(5-methyl-3-isoxazolyl)-4-(trifluoromethyl)benzenesulfonamide and 2-amino-N-(3,4dimethyl-5-isoxazolyl)-4-(trifluoromethyl)benzenesulfonamide as antibiotics. EP-A-510526 and EP-A-526708 were published respectively on 28 October 1992 and 10 February 1993 and disclose N-(4-pyrimidinyl)-benzenesulfonamide compounds having specific substituents at the 5-position on the pyrimidinyl ring.

For compound I, it is preferred that:
R¹ is phenyl or phenoxy, optionally substituted with alkyl, alkoxy, -NZ⁷Z⁸, halo, or hydroxy;
R² and R³ are each independently hydrogen, alkyl, or -NR⁷R⁸;
R⁴ and R⁵ are alkyl; and
R⁷, R⁸, Z⁷ and Z⁸ are each independently hydrogen, alkyl, or -C(O)alkyl.

Most preferred compounds are those wherein:
R¹ is phenyl or phenoxy, optionally substituted with alkyl, alkoxy, amino, alkylamino, dialkylamino, alkanoylamino, or hydroxy;
R² and R³ are each independently hydrogen, alkyl of 1 to 4 carbon atoms, amino, alkylamino, dialkylamino, or alkanoylamino; and
R⁴ and R⁵ are alkyl of 1 to 4 carbon atoms, especially methyl.

### Detailed Description of the Invention

Listed below are definitions of terms used in this specification. These definitions apply to the terms as used throughout this specification, individually or as part of another group, unless otherwise limited in specific instances.

The terms "alkyl" and "alkoxy" refer to straight or branched chain hydrocarbon groups having 1 to 10 carbon atoms. The terms "lower alkyl" and "lower alkoxy" refer to groups of 1 to 4 carbon atoms, which are preferred.

The term "aryl" or "ar-" refers to phenyl, naphthyl, and biphenyl.

The term "alkenyl" refers to straight or branched chain hydrocarbon groups of 2 to 10 carbon atoms having at least one double bond. Groups of two to four carbon atoms are preferred.

The term "alkynyl" refers to straight or branched chain groups of 2 to 10 carbon atoms having at least one triple bond. Groups of two to four carbon atoms are preferred.

The term "alkylene" refers to a straight chain bridge of 1 to 5 carbon atoms connected by single bonds (e.g., -(CH₂)ₓ- wherein x is 1 to 5), which may be substituted with 1 to 3 lower alkyl groups.

The term "alkenylene" refers to a straight chain bridge of 2 to 5 carbon atoms having one or two double bonds that is connected by single bonds and may be substituted with 1 to 3 lower alkyl groups. Exemplary alkenylene groups are -CH=CH-CH=CH-, -CH₂-CH=CH-, -CH₂-CH=CH-CH₂-, -C(CH₃)₂CH=CH-, and -CH(C₂H₅)-CH=CH.

The term "alkynylene" refers to a straight chain bridge of 2 to 5 carbon atoms that has a triple bond therein, is connected by singe bonds, and may be substituted with 1 to 3 lower alkyl groups. Exemplary alkynylene groups are -C≡C-, -CH₂-C≡C-, -CH(CH₃)-C≡C-, and -C≡C-CH(C₂H₅)CH₂-.

The term "alkanoyl" refers to groups of the formula -C(O)alkyl.

The terms "cycloalkyl" and "cycloalkenyl" refer to cyclic hydrocarbon groups of 3 to 8 carbon atoms.

The terms "halogen" and "halo" refer to fluorine, chlorine, bromine and iodine.

The compounds of formula I form salts which are also within the scope of this invention. Pharmaceutically acceptable (i.e., non-toxic, physiologically acceptable) salts are preferred, although other salts are also useful, e.g, in isolating or purifying the compounds of this invention.

The compounds of formula I may form salts with alkali metals such as sodium, potassium and lithium, with alkaline earth metals such as calcium and magnesium, with organic bases such as dicyclohexylamine, benzathine, N-methyl-D-glucamide and hydrabamine, and with amino acids such as arginine and lysine. Such salts may be obtained by reacting compound I with the desired ion in a medium in which the salt precipitates or in an aqueous medium followed by lyophilization.

When the R¹ to R⁵ substituents comprise a basic moiety, such as amino or substituted amino, compound I may form salts with a variety of organic and inorganic acids. Such salts include those formed with hydrochloric acid, hydrogen bromide, methanesulfonic acid, sulfuric acid, acetic acid, maleic acid, benzenesulfonate, toluenesulfonate, and various other sulfonates, nitrates, phosphates, borates, acetates, tartrates, maleates, citrates, succinates, benzoates, ascorbates and salicylates. Such salts may be formed by reacting compound I in an equivalent amount of the acid in a medium in which the salt precipitates or in an aqueous medium followed by lyophilization.

In addition, when the R¹ to R⁵ substituents comprise a basic moiety such as amino, zwitterions ("inner salts") may be formed.

Certain of the R¹ to R⁵ substituents of compound I may contain asymmetric carbon atoms. Such compounds of formula I may exist, therefore, in enantiomeric and diasteromeric forms and in racemic mixtures thereof. All are within the scope of this invention.

The compounds of formula I are antagonists of ET-1, ET-2, and/or ET-3 and are useful in treatment of all endothelin-dependent disorders. They are thus useful as antihypertensive agents. By the administration of a composition having one (or a combination) of the compounds of this invention, the blood pressure of a hypertensive mammalian (e.g., human) host is reduced.

The compounds of the present invention are also useful in the treatment of disorders related to renal, glomerular, and mesangial cell function, including chronic renal failure, glomerular injury, renal damage secondary to old age, nephrosclerosis (especially hypertensive nephrosclerosis) and nephrotoxicity (including nephrotoxicity related to imaging and contrast agents). The compounds of this invention may also be useful in the treatment of disorders related to paracrine and endocrine function.

The compounds of the present invention are also useful in the treatment of endotoxemia or endotoxin shock.

The compounds of the present invention are also useful as anti-ischemic agents for the treatment of, for example, heart, renal and cerebral ischemia.

In addition, the compounds of this invention may also be useful as anti-arrhythmic agents; anti-anginal agents; anti-fibrillatory agents; anti-asthmatic agents; therapy for myocardial infarction; therapy for peripheral vascular disease (e.g., Raynaud's disease); anti-atherosclerotic agents; treatment of cardiac hypertrophy (e.g., hypertrophic cardiomyopathy); treatment of pulmonary hypertension; additives to cardioplegic solutions for cardiopulmonary bypasses; adjuncts to thrombolytic therapy; treatment of central nervous system vascular disorders, such as stroke, migraine, and subarachnoid hemorrhage; treatment of central nervous system behavioral disorders; treatment of gastrointestinal diseases, such as ulcerative colitis and Crohn's disease; anti-diarrheal agents; regulation of cell growth; and treatment of hepatoxicity and sudden death.

The compounds of this invention can also be formulated in combination with endothelin converting enzyme (ECE) inhibitors, such as phosphoramidon; platelet activating factor (PAF) antagonists; angiotensin II (All) receptor antagonists; renin inhibitors; angiotensin converting enzyme (ACE) inhibitors such as captopril, zofenopril, fosinopril, ceranapril, alacepril, enalapril, delapril, pentopril, quinapril, ramipril, lisinopril, and salts of such compounds; neutral endopeptidase (NEP) inhibitors; calcium channel blockers; potassium channel activators; beta-adrenergic agents; antiarrhythmic agents; diuretics, such as chlorothiazide, hydrochlorothiazide, flumethiazide, hydroflumethiazide, bendroflumethiazide, methylchlorothiazide, trichloromethiazide, polythiazide or benzothiazide as well as ethacrynic acid, tricrynafen, chlorthalidone, furosemide, musolimine, bumetanide, triamterene, amiloride and spironolactone and salts of such compounds; thrombolytic agents such as tissue plasminogen activator (tPA), recombinant tPA, streptokinase, urokinase, prourokinase, and anisoylated plasminogen streptokinase activator complex (APSAC). If formulated as a fixed dose, such combination products employ the compounds of this invention within the dosage range described below and the other pharmaceutically active agent within its approved dosage range. The compounds of this invention may also be formulated with or useful in conjunction with antifungal and immunosuppressive agents such as amphotericin B, cyclosporins and the like to counteract the glomerular contraction and nephrotoxicity secondary to such compounds. The compounds of this invention may also be used in conjunction with hemodialysis.

The compounds of the invention can be administered orally or parenterally to various mammalian species known to be subject to such maladies, e.g., humans, in an effective amount within the dosage range of about 0.1 to about 100 mg/kg, preferably about 0.2 to about 50 mg/kg and more preferably about 0.5 to about 25 mg/kg (or from about 1 to about 2500 mg, preferably from about 5 to about 2000 mg) in single or 2 to 4 divided daily doses.

The active substance can be utilized in a composition such as tablet, capsule, solution or suspension containing about 5 to about 500 mg per unit dosage of a compound or mixture of compounds of formula I or in topical form for wound healing (0.01 to 5% by weight compound of formula I, 1 to 5 treatments per day). They may be compounded in a conventional manner with a physiologically acceptable vehicle or carrier, excipient, binder, preservative, stabilizer, flavor, or with a topical carrier such as Plastibase (mineral oil gelled with polyethylene) as called for by accepted pharmaceutical practice.

The compounds of the invention may also be administered topically to treat peripheral vascular diseases and as such may be formulated as a cream or ointment.

The compounds of formula I can also be formulated in compositions such as sterile solutions or suspensions for parenteral administration. About 0.1 to 500 milligrams of a compound of formula I is compounded with a physiologically acceptable vehicle, carrier, excipient, binder, preservative, stabilizer, in a unit dosage form as called for by accepted pharmaceutical practice. The amount of active substance in these compositions or preparations is such that a suitable dosage in the range indicated is obtained.

The compounds of the present invention may be prepared as follows.

An amine 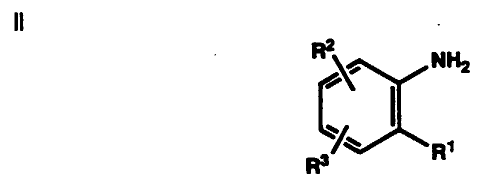 is treated with an acid (e.g., hydrochloric acid) and sodium nitrite in a solvent (e.g., water, acetic acid) at about -20 to 0°C, followed by sulfur dioxide, and a copper (I) salt (e.g., copper (I) chloride) in a solvent (e.g., acetic acid) at about 5 to 30°C to form a sulfonyl halide 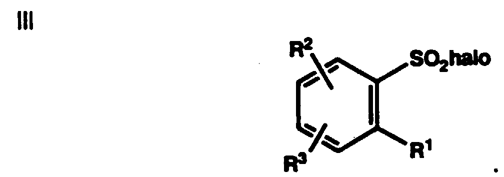 Sulfonyl halide III is coupled with an isoxazolamine 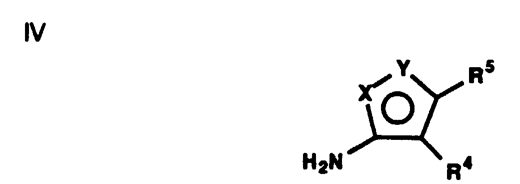 in an anhydrous organic solvent (e.g., pyridine) to form compound I.

Compound I wherein R¹, R² or R³ is aryl may be prepared by metal (e.g., palladium (0)) catalyzed coupling of the associated halo compound I (wherein R¹, R² or R³ is halogen) with aryl metalloids (i.e., aryl derivatives of tin, silicon and boron, such as phenylboronic acid). See, for example, the procedures of Example 4. A phenylboronic acid may be prepared by treating an aryl halide with n-butyllithium or magnesium tumings in tetrahydrofuran, and adding trimethylborate and finally aqueous hydrochloric acid. Alternatively, a phenylboronic acid may be prepared by adding a phenyl magnesium bromide to a solution of trimethyl borate and quenching with aqueous hydrochloric acid. Depending on the desired result, certain phenylboronic acids may be prepared by mixing an aryldihalide with palladium (0) and adding a grignard reagent to prepare a desired aryl halide before treating the aryl halide as described above.

Also depending on the desired result, certain phenylboronic acids may be prepared by ortho lithiation of a subsituted benzene. Treatment of the substituted benzene with, for example, n-butyl lithium removes an ortho proton. Subsequent treatment with B(OCH₃)₃ and HCI results in an ortho-substituted phenylboronic acid.

There are several other altematives for preparing a compound of the formula I wherein specifically R₁ is aryl. For example, a compound of the formula I may be prepared by metal (e.g., palladium (0)) catalyzed coupling of a halobenzenesulfonamide with a phenylboronic acid. The resulting biphenyl sulfonamide is reacted with a haloisoxazole and a base (e.g., Cs₂CO₃).

Alternatively, a halobenzene sulfonyl chloride may be reacted with a pyrrole. The product is coupled, using a metal (e.g., palladium(0)) catalyst, with a substituted phenylboronic acid, treated with a base (e.g., NaOH) and then PCl₅, and finally treated with an isoxazolamine of the formula IV.

Compounds of the formula I may also be prepared by treating a halobenzene, substituted in the meta position, with ClSO₃H. The resulting substituted halobenzene sulfonyl chloride is then treated as described above.

For compounds wherein any of R¹ to R⁵ comprise reactive functionalities, the reactants may be treated with protecting agents prior to coupling. The amine portion of the sulfonamide core may also need to be protected when different R¹, R² and R³ groups are added. Suitable protecting agents and procedures for use thereof are generally known in the art. Exemplary protecting groups are benzyl, halocarbobenzyloxy and tosyl for hydroxyl; carbobenzyloxy, halocarbobenzyloxy, acetyl, benzoyl and methoxyethoxymethyl for amino. The sulfonamide nitrogen may be protected methoxyethoxymethyl, trimethylsilylethoxymethyl and t-butyl. Protecting groups may be removed from the resulting protected analogues of compound I by treatment with one or more deprotecting agents. Suitable deprotecting agents and procedures for use thereof are generally known in the art.

To form compound I wherein one or more of R¹ to R³ is -NR⁷R⁸ and R⁷ and/or R⁸ is -C(O)R⁶, the associated nonacyl sulfonic acid 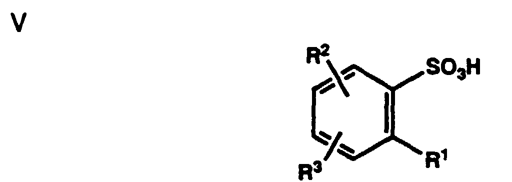 is treated with water and an alkali metal hydroxide (e.g., sodium hydroxide) to form a sulfonic acid salt 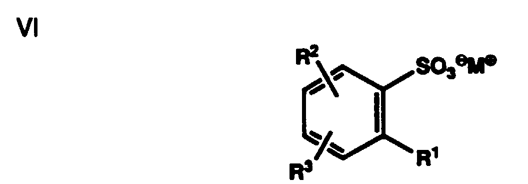 wherein M⁺ is a lithium, sodium or potassium ion. Salt VI is treated with an acylating agent (e.g., acetic anhydride) at about 90 to 110°C in either the acylating agent as solvent or in an anhydrous organic solvent (e.g., pyridine) to form the associated acylamine of formula VI, wherein one or more of R¹, R² and R³ is -N R⁷R⁸ and at least one of R⁷ and R⁸ is -C(O)R⁶. Acylamine VI is then treated with a halosulfonic acid solution (e.g., chlorosulfonic acid) or with another halogenating agent (e.g., phosphorus pentachloride, thionyl chloride) at about 0°C to 80°C to form an acyl-sulfonic halide III, which is coupled with isoxazolamine IV as described above to form compound I wherein at least one of R¹, R² and R³ is -NR⁷R⁸ and at least one of R⁷ and R⁸ is -C(O)R⁶.

To form compound I wherein one or more of R¹ to R³ is alkoxy, the associated sulfonic acid V wherein one or more of R¹ to R³ is hydroxy may be treated with an alkylating agent (e.g., dimethylsulfate) and an alkali metal hydroxide (e.g., sodium hydroxide) in an aqueous/organic solvent mixture (e.g., water/ethanol). The resulting alkoxy sulfonic add salt VI may be used as described above to form compound I.

Monoamines of formula I (for example having -NR⁷R⁸ wherein one of R⁷ and R⁸ is hydrogen) are prepared from the assodated free amine (for example wherein R⁷ and R⁸ are both hydrogen). The free amine is treated with (1) a ketone or aldehyde (e.g., acetone), (2) a reducing agent (e.g., sodium cyanoborohydride) or hydrogen gas (H₂) and a catalyst (e.g., palladium on carbon), and (3) an acid (e.g., acetic acid, hydrochloric acid) in an organic solvent (e.g., methanol) to form the associated monoamine compound I. Diamines of formula I, of course, may be similarly prepared. Additionally, the monoamines may be acylated.

Monoamines of formula I (for example having -NR⁷R⁸ wherein one of R⁷ and R⁸ is hydrogen) may also be prepared from the associated acylamine by treatment with a reducing agent, for example, borane.

The invention will now be further described by the following working examples, which are preferred embodiments of the invention.

### Example 1

### N-(3,4-Dimethyl-5-isoxazolyl)[1,1'-biphenyl]-2-sulfonamide

### A. 2-Phenylbenzenesulfonyl chloride

2-Aminobiphenyl (5.08 g, 30 mmol) was added in one portion to a mixture of concentrated hydrochloric acid (10 mL) and glacial acetic acid (3 mL) in a beaker equipped with a mechanical stirrer. The thick pink hydrochloride salt was cooled in a dry ice-ethanol bath to -10°C. A solution of sodium nitrite (2.24 g, 32.5 mmol) in water (3.5 mL) was added dropwise at a rate such that the temperature did not exceed -5°C. This mixture was stirred for 45 min maintaining the temperature between -10°C and -5°C. In a separate beaker, sulfur dioxide gas was bubbled through 30 mL of glacial acetic acid under vigorous stirring for 20 minutes. Copper(I) chloride (0.75 g) was added to this solution and bubbling of sulfur dioxide gas was continued until the yellow-green suspension became blue-green and most of the solids dissolved (about 30 minutes). This mixture was cooled to 10°C in an ice bath with stirring and to it was added the diazotization mixture in portions over 30 minutes, after which period the ice-bath was removed and the mixture was allowed to warm to room temperature. The green mixture was stirred for an additional 30 minutes and poured into ice-water (100 mL, 1:1) and the precipitated gummy solid was extracted with ether (3 x 75 mL). The combined extracts were washed with saturated sodium bicarbonate solution until neutral and washed with water (2 x 50 mL), dried (magnesium sulfate) and concentrated under vacuum to yield 5.0 g (66%) of compound A as a light brown solid.

### B. N-(3,4-Dimethyl-5-isoxazolyl)[1,1'-biphenyl]-2-sulfonamide

To a solution of 3,4-dimethyl-5-isoxazolamine (1.32 g, 11.8 mmol) in dry pyridine (5 mL) was added compound A (2.5 g, 9.8 mmol) in portions over 15 minutes. More pyridine was added to bring the total volume to approximately 10 mL. The resulting dark red-brown solution was stirred overnight at room temperature. The reaction mixture was added dropwise to ice-water (100 mL, 1:1) and the resulting tarry precipitate was filtered through Celite®, and the filtrate was acidified with 6 N hydrochloric acid to pH 2. A brown solid was filtered, washed with water and dried. This solid (1.4 g) was purified by flash chromatography over silica gel with ethyl acetate:hexanes (1:1) ) to give 1.1 g (34%) of Example 1 as a yellow solid.
Melting point: 171-173°C.

| Analysis for C₁₇H₁₆N₂O₃S (328.4) | | | | | | | | |
|---|---|---|---|---|---|---|---|---|
| Calc'd: | C, | 62.18; | H, | 4.91; | N, | 8.53; | S, | 9.76. |
| Found: | C, | 62.27; | H, | 4.92; | N, | 8.39; | S, | 10.02. |

### Example 2

### N-(3,4-Dimethyl-5-isoxazolyl)-2-bromobenzene-sulfonamide

To a solution of 3.0 g (11.74 mmol) of 2-bromobenzenesulfonyl chloride in 10 mL of pyridine was added 1.32 g (11.74 mmol) of 3,4-dimethyl-5-isoxazolamine. The mixture was stirred at room temperature under argon ovemight, added to 150 mL of ice water and filtered. The filtrate was acidified to pH 2 using 6 N aqueous hydrochloric acid and the grey solid was filtered and dried. The solid was crystallized from methanol/water to afford 4.0 g (greater than 100%) of Example 2 as tan crystalline needles.
Melting point: 125-126°C.

| Analysis for C₁₁H₁₁BrN₂O₃S | | | | | |
|---|---|---|---|---|---|
| Calc'd: | C, 39.89; | H, 3.35; | N, 8.46; | S, 9.68; | Br, 24.13. |
| Found: | C, 39.32; | H, 3.35; | N, 8.21; | S, 9.52; | Br, 24.08. |

### Example 3

### N-(3,4-Dimethyl-5-isoxazolyl)-2-phenoxybenzenesulfonamide

### A. 2-Phenoxybenzenesulfonyl chloride

To a solution of 6.0 g (32.4 mmol) of 2-phenoxyaniline in 15 mL of concentrated hydrochloric acid and 5 mL of glacial acetic acid at -5°C was added a solution of sodium nitrite (2.35 g, 34 mmol) in 5 mL of water dropwise over 15 minutes. The solution was stirred at -5°C for an additional 1 hour. During the diazotization, sulfur dioxide was bubbled through 30 mL of glacial acetic acid until it was saturated (about 10 minutes). Cuprous chloride (1.5 g) was then added and the introduction of sulfur dioxide was continued (about 20 minutes) until the yellow-green suspension became blue-green. The mixture was cooled to 10°C and the solution containing the diazonium salt was added in portions over 15 minutes. The green reaction mixture was warmed to room temperature and stirred for an additional 1 hour. Water (150 mL) was added and the solution was extracted with ether (3 x 100 mL). The combined ether extracts were repeatedly washed with 5% aqueous sodium hydrogen carbonate (5 x 150 mL) until neutral and then with water (150 mL) and dried and evaporated to give 2.75 g of compound A as a brown syrup.

### B. N-(3,4-Dimethyl-5-isoxazolyl)-2-phenoxybenzenesulfonamide

To a solution of 2.7 g of crude compound A (10 mmol) in 15 mL of pyridine was added 0.79 g (7 mmol) of 3,4-dimethyl-5-isoxazolamine, and the solution was stirred at room temperature overnight. The solution was diluted with 150 mL of ice water and the residual gum (2.5 g) was filtered. The filtrate was acidified to pH 2 using 6 N aqueous hydrochloric acid and the solid was filtered (0.23 g) and chromatographed on 10 g of silica using 1:1 hexanes/ethyl acetate to provide 0.16 g (7%) of Example 3 as a white crystalline solid.
Melting point: 181-182°C.

| Analysis for C₁₇H₁₆N₂O₄S | | | | |
|---|---|---|---|---|
| Calc'd: | C, 59.29; | H, 4.68; | N, 8.13; | S, 9.31. |
| Found: | C, 59.15; | H, 4.57; | N, 8.08; | S, 9.35. |

### Example 4

### 3'-Amino-N-(3,4-dimethyl-5-isoxazolyl)[1,1'-biphenyl]-2-sulfonamide

### A. 2-Bromo-N-(3,4-dimethyl-5-isoxazolyl)-N'-(methoxyethoxymethyl)benzenesulfonamide

To a solution of 1.1 g (3.33 mmol) of 2-bromo-N-(3,4-dimethyl-5-isoxazolyl)benzenesulfonamide (Example 2) in 15 mL of tetrahydrofuran at room temperature under argon was added 0.19 g (4.8 mmol) of sodium hydride (60% suspension in mineral oil) in portions, and the solution was stirred at room temperature for 10 minutes. Methoxyethoxymethyl chloride (0.55 g, 4.4 mmol) was then added and the solution was stirred overnight. The mixture was concentrated and diluted with 30 mL of water and extracted with 3 x 40 mL of ethyl acetate. The combined organic extracts were washed with 50 mL of brine, dried and evaporated to provide 1.2 g (87%) of compound A as a brown gum.

### B. 3'-Amino-N-(3,4-dimethyl-5-isoxazolyl)-N'-(methoxyethoxymethyl)[1,1'-biphenyl]-2-sulfonamide

To a solution of 1.12 g (2.67 mmol) of compound A and 0.15 g (0.13 mmol) of tetrakis(triphenylphosphine)palladium(0) in 15 mL of benzene under argon, 7.6 mL of 2 M aqueous sodium carbonate was added, followed by 0.46 g (2.93 mmol) of 3-aminophenylboronic acid in 5 mL of 95% ethanol. The mixture was refluxed overnight, diluted with 35 mL of water, and extracted with 3 x 35 mL of ethyl acetate. The combined organic extracts were washed once with 35 mL of brine, dried and evaporated. The residue was chromatographed on 120 g of silica gel using hexanes/ethyl acetate (1:2) to afford 0.75 g (65%) of compound B as a gum.

### C. 3'-Amino-N-(3,4-dimethyl-5-isoxazolyl)[1,1'-biphenyl]-2-sulfonamide

To a solution of 0.72 g (1.7 mmol) of compound B in 10 mL of 95% ethanol, 10 mL of 3 N aqueous hydrochloric acid was added and the solution was refluxed for 7 hours. The mixture was concentrated, diluted with 40 mL of water and neutralized to pH 7 using aqueous sodium hydrogen carbonate. The mixture was extracted with 4 x 50 mL of ethyl acetate and the combined organic extracts were washed once with 50 mL of brine, dried and evaporated. The residue was chromatographed on 25 g of silica using methylene chloride:methanol (97:3) and triturated with ether/hexanes to afford 86 mg of Example 4 as a tan solid.
Melting point: 157-160° C.

| Analysis for C₁₇H₁₇N₃O₃S-0.1 C₆H₁₄ | | | | |
|---|---|---|---|---|
| Calc'd: | C, 60.05; | H, 5.27; | N, 11.94; | S, 9.11. |
| Found: | C, 59.83; | H, 5.11; | N, 11.55; | S, 8.69. |

### Example 5

### 2-Fluoro-N-(3,4-dimethyl-5-isoxazolyl)benzenesulfonamide

To a solution of 2.88 g (25.7 mmol) of 3,4-dimethyl-5-isoxazolamine in 15 mL of pyridine was added 5.0 g (25.7 mmol) of 2-fluorobenzenesulfonyl chloride. The mixture was stirred at room temperature overnight, poured into 100 mL of ice water and the resulting mixture was filtered. The filtrate was acidified to pH 2 using 6 N aqueous hydrochloric acid and the solid was filtered and dried to provide 3.2 g (46%) of Example 5 as a tan solid.
Melting point: 122-124°C.

| Analysis for C₁₁H₁₁FN₂O₃S | | | | | |
|---|---|---|---|---|---|
| Calc'd: | C, 48.88; | H, 4.10; | N, 10.36; | S, 11.86; | F, 7.03. |
| Found: | C, 48.93; | H, 3.77, | N, 10.38; | S, 12.10; | F, 6.70. |

### Example 6

### N-[3-[[(3,4-Dimethyl-5-isoxazolyl)amino]sulfonyl]-4-methylphenyl]acetamide

### A. 5-Amino-2-methylbenzenesulfonic acid, sodium salt

To a suspension of 5-amino-2-methylbenzenesulfonic acid (25 g, 134 mmol) in water (100 mL) was added 4 N sodium hydroxide (34 mL). The resulting clear brown solution was evaporated and the remaining brown solid was washed several times with ether and dried to afford 31.3 g of compound A as a brown solid (greater than 100%).

### B. 5-Acetylamino-2-methylbenzenesulfonic acid, sodium salt

A suspension of crude compound A (25 g, about 107 mmol) in acetic anhydride (100 mL) was heated at 100°C for 3 hours, allowed to stand overnight at room temperature and evaporated. The residual gummy brown solid was suspended in ether, the suspension was filtered and the solid was washed twice with ether to afford 32.3 g of. compound B as a tan solid (greater than 100%), which appeared to be hygroscopic.

### C. 5-Acetylamino-2-methylbenzenesulfonyl chloride

A mixture of Compound B (18 g, about 71.6 mmol) and phosphorus pentachloride (30 g, 143 mmol) was heated at 75°C with stirring for 2.25 hours, during which time the solids liquefied to a brown gum. The mixture was cooled and the dark brown semi-solid was poured into ice water (400 mL). The brown solid that formed was filtered, washed with water and dissolved in methylene chloride. The organic solution was washed with water and dried (magnesium sulfate) and evaporated to afford 14.4 g of brown foamy gum. This material was dissolved in methylene chloride and passed through a pad of silica using 50% ethyl acetate/hexanes to afford 10.2 g of brown gum. Flash chromatography on silica with 60% ethyl acetate/hexanes afforded 2.01 g of compound C (11%) as a light yellow oil that crystallized on standing.

### D. N-[3-[[(3,4-Dimethyl-5-isoxazolyl)amino]sulfonyl]-4-methylphenyl]acetamide

A solution of Compound C (1.02 g, 4.12 mmol), 3,4-dimethyl-5-isoxazolamine (0.55 g, 4.94 mmol) and dimethylaminopyridine (0.10 g, 0.82 mmol) in 4 mL of pyridine was heated at 70°C for 2.25 hours, cooled and poured onto iced dilute hydrochloric acid. The resulting tan solid was filtered, rinsed with water and dissolved in 10% isopropanol/methylene chloride. The solution was dried (magnesium sulfate) and evaporated to afford 1.09 g of a brown foamy gum which was flash-chromatographed on silica (2%, then 3%, then 5%, then 10% methanol/methylene chloride) to provide 0.37 g of clean Example 6 as a white foam. There was also obtained 0.43 g of impure Example 6. Crystallization of the clean material from aqueous ethanol afforded 0.25 g of Example 6 (19%) as light tan crystals.
Melting point: 203-204°C.

| Analysis for C₁₄H₁₇N₃O₄S | | | | |
|---|---|---|---|---|
| Calc'd: | C, 52.00; | H, 5.30; | N, 12.99; | S, 9.91. |
| Found: | C, 51.81; | H, 5.31; | N, 12.86; | S, 9.94. |

### Example 7

### 5-Amino-N-(3,4-Dimethyl-5-isoxazolyl)-2-methylbenzenesulfonamide

A solution of crude Example 6 (0.40 g, 1.24 mmol) in 4 N sodium hydroxide (4 mL, 16 mmol) and methanol (1 mL) was heated at 65°C for 4.5 hours, cooled and the methanol evaporated. The residue was extracted with ether and the aqueous solution was acidified to pH 2.5 with concentrated hydrochloric acid and extracted twice.with 10% isopropanol/methylene chloride. The organic phase was dried (magnesium sulfate) and evaporated to afford 0.37 g of a yellow oil that crystallized on standing. Recrystallization from aqueous ethanol afforded 0.24 g of Example 7 (69%) as light tan needles.
Melting point: 204-205°C.

| Analysis for C₁₂H₁₅N₃ O₃ S | | | | |
|---|---|---|---|---|
| Calc'd: | C, 51.23; | H, 5.37; | N, 14.94; | S, 11.40. |
| Found: | C, 51.52; | H, 5.37; | N, 14.92; | S, 11.57. |

### Example 8

### N-(3,4(Dimethyl-5-isoxazolyl)-2-(1-methylethyl)benzenesulfonamlde

### A. 2-Isopropylbenzenesulfonate, sodium salt

To a solution of 30% hydrogen peroxide (10 mL) in glacial acetic acid (10 mL) at 50°C was added a solution of 2-isopropylthiophenol (1 g, 6.58 mmol) in 5 mL of glacial acetic acid over 1 hour. After the addition was completed, the reaction was heated to 60°C for 1 hour. The reaction was concentrated in vacuo and the residue was lyophilized from water to afford 1.3 g of a white solid. The solid (theoretically 6.5 mmol) was dissolved in 5 mL of water and 4 N aqueous sodium hydroxide (1.62 mL, 6.5 mmol) was added. The solution was lyophilized to afford 1.4 g (100 %) of compound A as a white solid.
MS (M+NH₄)⁺ 218; (M-H)-199.

### B. 2-Isopropylbenzenesulfonyl chloride

To a slurry of compound A (600 mg, 2.50 mmol) in chloroform (20 mL) was added chlorosulfonic acid (0.33 mL, 5.0 mmol) dropwise to maintain the reaction temperature below 50°C. The reaction was heated to 60°C ovemight, cooled to room temperature and poured into ice-water. The aqueous solution was extracted with chloroform (three times). The combined organic phases were dried over sodium sulfate. The solvent was removed in vacuo to afford 410 mg (74.5%) of compound B.

### C. N-(3,4-Dimethyl-5-isoxazolyl)-2-(1-methylethyl)benzenesulfonamide

A solution of compound B (410 mg, 1.88 mmol), dimethylaminopyridine (60 mg, 0.49 mmol), and 3,4-dimethyl-5-isoxazolamine (230 mg, 2.06 mmol) in pyridine (8 mL) was heated in an oil bath at 70°C for 2 hours. The reaction was poured onto iced 10% hydrochloric acid. The mixture was extracted with ethyl acetate (three times) and the combined organic phases were extracted with 10% aqueous sodium hydrogen carbonate. The aqueous solution was acidified to pH 3 and extracted with ethyl acetate (three times). The combined organic phases were washed with saturated sodium chloride, dried over sodium sulfate and evaporated. The residue was applied to a silica gel column (20 x 130 mm) and eluted with ethyl acetate:hexanes (1:1). The enriched product fractions were combined and evaporated. The residue was applied to three 20 x 20 chromatographic thick plates. The plates were eluted with 1:1 ether:hexanes. The desired bands were cut and extracted with ethyl acetate. The silica gel was filtered and the organic solvent was evaporated to afford 192.2 mg (35%) of Example 8 as a yellow semi-solid.
MS: (M+H)⁺ 295.

| Analysis for C₁₄H₁₈N₂O₃S-0.58 H₂O | | | | |
|---|---|---|---|---|
| Calc'd: | C, 55.15; | H, 6.34; | N, 9.19; | S, 10.52. |
| Found: | C, 55.22; | H, 6.42; | N, 9.12; | S, 10.72. |

### Example 9

### N-(3,4-Dimethyl-5-isoxazolyl)-2-nitro-benzenesulfonamide

To a solution of 4.04 g (36 mmol) of 3,4-dimethyl-5-isoxazolamine in 15 mL of pyridine, 8.0 g (36 mmol) of 2-nitrobenzenesulfonyl chloride was added and the solution was stirred at room temperature ovemight. The mixture was poured into 100 mL of ice water and filtered. The filtrate was acidified to pH 2 using 6 N aqueous hydrochloric acid and the mixture was extracted with 4 x 125 mL of ethyl acetate. The combined organic extracts were washed with 75 mL of brine, dried and evaporated to provide 9.1 g of a dark brown residue. This material was chromatographed on silica gel using hexanes/ethyl acetate (2:1) to provide 0.5 g of Example 9 as a light yellow solid.
Melting point: 91-94°C.

| Analysis for C₁₁H₁₁N₃O₅S | | | | |
|---|---|---|---|---|
| Calc'd: | C, 44.44; | H, 3.73; | N, 14.13; | S, 10.78. |
| Found: | C, 44.75; | H, 3.69; | N, 14.01; | S, 11.06. |

### Example 10

### 2-Amino-N-(3,4-dimethyl-5-isoxazolyl)benzenesulfonamide

To a suspension of 135 mg of 10% palladium on carbon in 20 mL of methanol under argon, 0.9 g (3.03 mmol) of N-(3,4-dimethyl-5-isoxazolyl)-2-nitrobenzenesulfonamide (Example 9) in 20 mL of methanol was added The solution was hydrogenated with a balloon filled with hydrogen for 90 minutes. The mixture was filtered through Celite® and the filtrate was concentrated to afford 0.9 g of a gum. This material was chromatographed on silica initially with 9:1 methylene chloride:methanol and then with 1:1 hexanes:ethyl acetate to provide 0.2 g (24%) of Example 10 as a white solid.
Melting point: 116-118°C.

| Analysis for C₁₁H₁₃N₃O₃S | | | | |
|---|---|---|---|---|
| Calc'd: | C, 49.43; | H, 4.90; | N, 15.72; | S, 11.99. |
| Found: | C, 49.56; | H, 4.80; | N, 15.62; | S, 11.89. |

### Example 11

### N-(3,4-Dimethyl-5-isoxazolyl)-4'-methyl[1,1'-biphenyl]-2-sulfonamide

### A. 4'-Methyl-N-(3,4-dimethyl-5-isoxazolyl)-N-(methoxyethoxymethyl)[1,1'-biphenyl]-2 -sulfonamide

To a solution of 0.78 g (1.86 mmol) of compound A from Example 4 and 0.096 g (0.08 mmol) of tetrakis(triphenylphosphine)palladium(0) in 15 mL of benzene under argon, 8.0 mL of 2 M aqueous sodium carbonate was added followed by 0.38 g (2.79 mmol) of 4-methylphenylboronic acid in 10 mL of 95% ethanol. The mixture was refluxed ovemight and diluted with 50 mL of water and extracted with 3 x 100 mL of ethyl acetate. The combined organic extracts were washed once with 100 mL of brine and dried and evaporated. The residue was chromatographed on 100 g of silica gel using hexanes/ethyl acetate (2:1) to afford 0.65 g (81%) of compound A as a colorless gum.

### B. N-(3,4-Dimethyl-5-isoxazolyl)-4'-methyl[1,1'-biphenyl]-2-sulfonamide

To a solution of 0.56 g (1.3 mmol) of compound A in 10 mL of 95% ethanol, 10 mL of 3 N aqueous hydrochloric acid was added and the solution was refluxed for 18 hours. The mixture was concentrated and diluted with 25 mL of water. The mixture was extracted with 3 x 50 mL of ethyl acetate and the combined organic extracts were washed once with 50 mL of brine and dried and evaporated. Crystallization of the residue (0.41 g) from hexanes/ethyl acetate provided 0.37 g (83%) of Example 11 in two crops.
Melting point: 126-127°C.

| Analysis for C₁₈H₁₈N₂O₃S | | | | |
|---|---|---|---|---|
| Calc'd: | C, 63.14; | H, 5.30; | N, 8.18; | S, 9.36. |
| Found: | C, 63.03; | H, 5.29; | N, 8.07; | S, 9.34. |

### Example 12

### 2'-Amino-N-(3,4-dimethyl-5-isoxazolyl)-[1,1'-biphenyl]-2-sulfonamide

### A. 2'-Amino-N-(3,4-dimethyl-5-isoxazolyl)-N-[(2-methoxyethoxy)methyl][1,1'-biphenyl]-2 -sulfonamide

To a solution of compound A from Example 4 (0.5g, 1.19 mmol) and tetrakis(triphenylphosphine)palladium(0) (0.062g, 0.05 mmol) in 10 mL of benzene under argon, 4.0 mL of 2M aqueous sodium carbonate was added followed by 2-amino-phenylboronic acid (0.245 g, 1.79 mmol) in 5 mL of 95% ethanol. The mixture was refluxed for 10 hours, diluted with 50 mL of water and extracted with ethyl acetate (3 x 50 mL). The combined organic extracts were washed once with 50 mL of brine, dried and evaporated. The residue was chromatographed on 75g of silica gel using hexanes/ethyl acetate (2:1) to afford 0.39 g (76%) of compound A as a colorless gum.

### B. 2'-Amino-N-(3,4-dimethyl-5-isoxazolyl)-[1,1'-biphenyl]-2-sulfonamide

To a solution of compound A (0.35g, 0.81 mmol) in 10 mL of 95% ethanol, 10 mL of 3N aqueous hydrochloric acid was added and the solution was refluxed for 6 hours. The mixture was concentrated, diluted with 10 mL of water, neutralized with saturated aqueous sodium hydrogen carbonate and acidified to pH 4 using glacial acetic acid. The mixture was extracted with ethyl acetate (3 x 25 mL) and the combined organic extracts were washed once with 50 mL of brine, dried and evaporated. Chromatography of the residue on 50g silica gel using hexanes/ethyl acetate (1 : 1) provided 0.087 g of a gum. Repeated crystallizations from ethyl acetate:methanol:hexanes (1:1:20) afforded Example 12 as a light brown solid, m.p. 182-183°C.

| Analysis for C₁₇H₁₇N₃O₃S | | | | |
|---|---|---|---|---|
| Calc'd: | C, 59.46; | H, 4.99; | N, 12.24; | S, 9.34. |
| Found: | C, 59.17; | H, 5.04; | N, 11.87; | S, 9.73. |

### Example 13

### 3'-(Dimethylamino)-N-(3,4-dimethyl-5-isoxazolyl)[1,1'-biphenyl]-2-sulfonamide

To a solution of Example 4 (0.46g, 1.34 mmol) in methanol (15 mL), 37% aqueous formaldehyde (0.44 mL, 5.36 mmol) and glacial acetic acid (0.49g) were added with stirring. Sodium cyanoborohydride (0.34g, 5.36 mmol) was added over 10 minutes and the solution was stirred overnight. The mixture was concentrated to about 10 mL, diluted with water (40 ml) and extracted with ethyl acetate (3 x 35 mL). The combined organic extracts were washed with brine (50 mL), dried (magnesium sulfate) and evaporated. The gum (0.45g) thus obtained was chromatographed on 100g of silica gel using hexanes/ethyl acetate (3:1) to afford 0.21g (42%) of Example 13 as an off-white solid, m.p. 67-70°C.

| Analysis for C₁₉H₂₁N₃O₃S-0.25 H₂O | | | | |
|---|---|---|---|---|
| Calc'd: | C, 60.69; | H, 5.76; | N, 11.18; | S, 8.53. |
| Found: | C, 60.92; | H, 5.74; | N, 10.95; | S, 8.33. |

### Example 14

### N-(3,4-Dimethyl-5-isoxazolyl)-2-(trifluoromethyl)benzenesulfonamide

To a solution of 1.38g (12.26 mmol) of 3,4-dimethyl-5-isoxazolamine in 10 mL of pyridine, 3.0g (12.26 mmol) of 2-trifluoromethylbenzenesulfonyl chloride was added and the solution was stirred at room temperature under argon ovemight. The mixture was added to 100 mL of ice water and filtered. The filtrate was acidified to pH 2 using 6N aqueous hydrochloric acid and the resultant gum was filtered and chromatographed on silica gel (200g) using 3% methanol in methylene chloride to provide a colorless gum. This material was crystallized from hexanes/ethyl acetate to afford 2.0 g (51%) of Example 14 as white crystalline needles, m.p 99-100°C.

| Analysis for C₁₂H₁₁F₃N₂O₃S | | | | | |
|---|---|---|---|---|---|
| Calc'd: | C, 45.00; | H, 3.46; | N, 8.75; | S, 10.01; | F, 17.80. |
| Found: | C, 44.67; | H, 3.55; | N, 8.74; | S, 10.51; | F, 18.19. |

### Example 15

### 2-Chloro-N-(3,4-dimethyl-5-isoxazolyl)-6-methylbenzenesulfonamide

Example 15 was prepared from 3,4-dimethyl-5-isoxazolamine and 2-chloro-6-methylbenzenesulfonyl chloride as described for Example 14. Crystallization from methanol/water afforded Example 15 as white crystalline prisms, m.p 181-182°C.

| Analysis for C₁₂H₁₃ClN₂O₃S | | | | | |
|---|---|---|---|---|---|
| Calc'd: | C, 47.92; | H, 4.36; | N, 9.31; | S, 10.66; | Cl, 10.66. |
| Found: | C, 47.61; | H, 4.25; | N, 9.07; | S, 10.67; | Cl, 10.67. |

### Example 16

### 4'-(Dimethylamino)-N-(3,4-dimethyl-5-isoxazolyl)[1,1'-biphenyl]-2-sulfonamide

### A. 4'-(Dimethylamino)-N-(3,4-dimethyl-5-isoxazolyl)-N-[(2-methoxyethoxy)methyl][1,1'-biphenyl]-2 -sulfonamide

Compound A was prepared from 4-dimethylaminophenylboronic acid and compound A from Example 4 as described for compound A from Example 12. Chromatography on silica gel using 3:1 hexanes/ethyl acetate afforded compound A as a colorless gum.

### B. 4'-(Dimethylamino)-N-(3,4-dimethyl-5-isoxazolyl)[11',-biphenyl]-2-sulfonamide

Example 16 was prepared from compound A as described for Example 12, with refluxing for 8 hours. Before ethyl acetate extraction, the aqueous phase was taken to pH 6 using glacial acetic acid. Chromatography on silica gel using 2:1 hexanes/methylene chloride and crystallization from hexanes/ethyl acetate provided Example 16 as colorless prisms, m.p. 135-136°C.

| Analysis for C₁₉H₂₁N₃O₃S | | | | |
|---|---|---|---|---|
| Calc'd: | C, 61.44; | H, 5.70; | N, 11.31; | S, 8.63. |
| Found: | C, 61.26; | H, 5.55; | N, 11.15; | S, 8.99. |

### Example 17

### N-[2'-[[(3,4-Dimethyl-5-isoxazolyl)amino]sulfonyl][1,1'-biphenyl]-3-yl]acetamide

To a solution of Example 4 (0.3g, 0.87 mmol) in pyridine (5 mL), acetic anhydride (0.13g) was added and the mixture was stirred at room temperature overnight. The mixture was concentrated and diluted with water (30 mL) and extracted with ethyl acetate (3 x 25 mL). The combined organic extracts were washed with brine (30 mL), dried (magnesium sulfate) and evaporated. The white solid (0.31g) thus obtained was chromatographed on 75g of silica gel using hexanes/ethyl acetate (2:1) to afford 0.18g (54%) of Example 17 as a white solid, m.p. 168-171°C.

| Analysis for C₁₉H₁₉N₃O₄S | | | | |
|---|---|---|---|---|
| Calc'd: | C, 59.21; | H, 4.97; | N, 10.90; | S, 8.32. |
| Found: | C, 59.33; | H, 4.86; | N, 10.57; | S, 8.47. |

### Example 18

### N-(3,4-Dimethyl-5-isoxazolyl)-4'-propyl[1,1'-biphenyl]-2-sulfonamide

### A. 4-Propylphenylboronic Acid

To a solution of trimethylborate (2.6 g, 25 mmol) in 10 mL of ether at -78°C under argon, 4-propylphenyl magnesium bromide (1.7 M solution in ether, 14.7 mL, 25 mmol) was added over 15 min. After 30 min at -78°C, the solution was warmed to room temperature and stirred for 90 min. The reaction was quenched by the addition of 10% aqueous hydrochloric acid (75 mL) and after 10 min the solution was extracted with ether (3 x 100 mL). The combined ether extracts were extracted with 1 M sodium hydroxide (2 x 100 mL) and the aqueous extracts were acidified with dilute hydrochloric acid to pH 2 and extracted with ether (2 x 100 mL). The combined ether extracts were washed once with water (100 mL), dried and evaporated to afford 1.85g (45%) of compound A as a tan solid, m.p. 95-96°C.

### B. N-(3,4-Dimethyl-5-isoxazolyl)-N-[(2-methoxyethoxy)methyl]-4'-propyl[1,1'-biphenyl]-2 -sulfonamide

Compound B was prepared from compound A and compound A from Example 4 as described for compound A from Example 12. Chromatography on silica gel using hexanes/ethyl acetate (3:1) afforded compound B as a colorless gum.

### C. N-(3,4-Dimethyl-5-isoxazolyl)-4'-propyl(1,1'-biphenyl]-2-sulfonamide

To a solution of 0.70g (1.53 mmol) of compound B in 15 mL of 95% ethanol, 15 mL of 3N aqueous hydrochloric acid was added. The solution was refluxed for 11 hours, concentrated and diluted with 25 mL of water. The mixture was extracted with ethyl acetate (3 x 50 mL) and the combined organic extracts were washed once with 50 mL of brine, dried and evaporated. Chromatography on 100g of silica gel using 4:1 followed by 3:1hexanes/ethyl acetate (1L) provided 0.38g (67%) of Example 18 as a colorless gum.

| Analysis for C₂₀H₂₂S₂O₃S | | | | |
|---|---|---|---|---|
| Calc'd: | C, 64.84; | H, 5.99; | N, 7.56; | S, 8.65. |
| Found: | C, 64.52; | H, 5.98; | N, 7.26; | S, 8.30. |

### Example 19

### 2-(Dimethylamino)-N-(3,4-dimethyl-5-isoxazolyl)benzenesulfonamide

Example 19 was prepared from Example 10 as described for Example 13. Chromatography on silica gel using hexanes/ethyl acetate (4:1) afforded Example 19 as a colorless gum.

| Analysis for C₁₃H₁₇N₃O₃S | | | | |
|---|---|---|---|---|
| Calc'd: | C, 52.87; | H, 5.80; | N, 14.23; | S, 10.85. |
| Found: | C, 52.99; | H, 5.87; | N, 14.06; | S, 11.28. |

### Example 20

### 2'-(Dimethylamino)-N-(3,4-dimethyl-5-isoxazolyl)-[1,1'-biphenyl]-2-sulfonamide

### A. 2'-(Dimethylamino)-N-(3,4-dimethyl-5-isoxazolyl)-N-[(2-methoxyethoxy-methyl)[1,1'-biphenyl]-2-sulfonamide

To a solution of compound A from Example 12 (0.45g, 1.04 mmol) in 15 mL of methanol under argon, glacial acetic acid (1 mL) and 37% aqueous formaldehyde (0.25 mL, 3.13 mmol) were added. The solution was stirred for 15 minutes, sodium cyanoborohydride (0.20g, 3.13 mmol) in 5 mL of methanol was added dropwise over 15 minutes and the solution was stirred for 24 hours. The mixture was evaporated, water (25 mL) was added and the mixture was extracted with ethyl acetate (2 x 50 mL). The combined organic extracts were dried and evaporated to provide 0.39 g (81%) of compound A as a light brown gum which solidified on standing.

### B. 2'-(Dimethylamino)-N-(3,4-dimethyl-5-isoxazolyl)-11,1'-biphenyl]-2-sulfonamide

Example 20 was prepared from compound A as described for Example 12. Following chromatography on silica gel using 3:1 hexanes:ethyl acetate, crystallization from methylene chloride/hexanes (∼1:5) afforded Example 20 as colorless prisms, m.p. 148-150°C.

| Analysis for C₁₉H₂₁N₃O₃S | | | | |
|---|---|---|---|---|
| Calc'd: | C, 61.44; | H, 5.70; | N, 11.31; | S, 8.63. |
| Found: | C, 61.32; | H, 5.69; | N, 11.30; | S, 8.72. |

### Example 21

### N-(3,4-Dimethyl-5-isoxazolyl)-4'-(2-methylpropyl)-[1,1'-biphenyl]-2-sulfonamide

### A. 4-isobutyl-phenylboronic acid

To a suspension of 0.68g (28.15 mmol) of magnesium turnings in 50 mL of tetrahydrofuran under argon, a crystal of iodine was added and a solution of 4-bromo-isobutylbenzene (6.0g, 28.15 mmol) in 25 mL of tetrahydrofuran was added at such a rate that a gentle reflux was maintained. The mixture was refluxed for an additional 1 hour, cooled to room temperature and added in portions over 15 min to a solution of trimethylborate (2.93g, 28.15 mmol) in 50 mL of ether at -78°C under argon. After 30 min at -78°C, the solution was warmed to room temperature, stirred for 90 minutes and 10% aqueous hydrochloric acid (100 mL) was added. After 10 minutes, the solution was extracted with ether (3 x 100 mL) and the combined ether extracts were extracted with 1 M sodium hydroxide (3 x 100 mL). The aqueous extracts were acidified with dilute hydrochloric acid to pH 2 and extracted with ether (3 x 100 mL). The combined ether extracts were washed once with water (100 mL), dried and evaporated to afford 3.5 g of a white solid. Crystallization from ether/hexanes provided 2.3g (46%) of compound A as a white solid in two crops, m.p. 134-135°C.

### B. N-(3,4-Dimethyl-5-isoxazolyl)-N-[(2-methoxyethoxy)methyl]-[4'(2-methylpropyl)][1,1' -biphenyl]-2-sulfonamide

Compound B was prepared from compound A and compound A from Example 4 as described for compound A from Example 12. Chromatography on silica gel using hexanes/ethyl acetate (3:1) afforded compound B as a colorless gum.

### C. N-(3,4-Dimethyl-5-isoxazolyl)-4'-(2-methylpropyl)-[1,1'-biphenyl]-2-sulfonamide

Example 21 was prepared from compound B as described for Example 18. Chromatography on silica gel using 3:1 hexanes/ethyl acetate followed by crystallization from methylene chloride/hexanes provided Example 21 as colorless prisms, m.p. 126°C.

| Analysis for C₂₁H₂₄N₂O₃S | | | | |
|---|---|---|---|---|
| Calc'd: | C, 65.60; | H, 6.29; | N, 7.29; | S, 8.34. |
| Found: | C, 65.59; | H, 6.16; | N, 7.28; | S, 8.50. |

### Example 22

### 4'-Butyl-N-(3,4-dimethyl-5-isoxazolyl)[1,1'-biphenyl]-2-sulfonamide

### A. 4-Butyl-phenylboronic acid

To a solution of 1-bromo-4-butylbenzene (6.24 g, 29.3 mmol) in tetrahydrofuran (32 mL) and ether (96 mL) at -78°C, n-butyllithium (1.6 M in hexane, 21.9 mL, 35.1 mmol) was added dropwise. The mixture was stirred at -78°C for 30 minutes and was added over 20 minutes to a solution of trimethyl borate (6.1 g, 58.6 mmol) in ether (64 mL) at -78°C. The mixture was stirred at -78°C for 30 minutes and at room temperature overnight. 10% aqueous hydrochloric acid (150 mL) was added, the mixture was shaken for 10 minutes, the ether layer was separated and the aqueous layer was extracted with ether (100 mL). The combined organic phases were extracted with 1 N sodium hydroxide (3 x 100 mL ) and the combined aqueous extracts were washed once with ether, acidified to pH 1 with 6N hydrochloric acid and extracted with ether (3 x 100 mL). The combined organic phases were washed with water, dried (magnesium sulfate) and concentrated to give compound A (2.0 g, 38%).

### B. 4'-Butyl-N-(3,4-dimethyl-5-isoxazolyl)-N-[(2-methoxyethoxy)methyl][1,1'-biphenyl]-2-sulfonamide

Compound B was prepared from compound A and compound A from Example 4 as described for compound A from Example 12. Chromatography on silica gel using 40:1 methylene chloride/ethyl acetate afforded compound B as a colorless gum.

### C. 4'-Butyl-N-(3,4-dimethyl-5-isoxazolyl)[1,1'-biphenyl]-2-sulfonamide

Compound C was prepared from compound B as described for Example 18, with refluxing for 8 hours. Chromatography on silica gel using 3:1 hexanes/ethyl acetate followed by crystallization from methylene chloride/hexanes provided Example 22 as colorless crystals, m.p. 92-93°C.

| Analysis for C₂₁H₂₄N₂O₃S | | | | |
|---|---|---|---|---|
| Calc'd: | C, 65.60; | H, 6.29; | N, 7.29; | S, 8.34. |
| Found: | C, 65.35; | H, 6.23; | N, 7.29; | S, 8.68. |

### Example 23

### N-(3,4-Dimethyl-5-isoxazolyl)-2-(1-naphthalenyl)benzenesulfonamide

### A. N-(3,4-Dimethyl-5-isoxazolyl)-N-[(2-methoxyethoxy)methyl]-2-(1-naphthalenyl) benzenesulfonamide

Compound A was prepared from 1-naphthaleneboronic acid and compound A from Example 4 as described for compound A from Example 12, with refluxing for 3.5 hours. Chromatography on silica gel using hexanes/ethyl acetate (3:1) afforded compound A as a colorless gum.

### B. N-(3,4-Dimethyl-5-isoxazolyl)-2-(1-naphthalenyl)benzenesulfonamide

Compound B was prepared from compound A as described for Example 18, using 6N hydrochloric acid and refluxing for 3 hours. Chromatography on silica gel using 3:1 hexanes/ethyl acetate followed by crystallization from methylene chloride/hexanes provided Example 23 as colorless prisms, m.p. 182-183°C.

| Analysis for C₂₁H₁₈N₂O₃S | | | | |
|---|---|---|---|---|
| Calc'd: | C, 66.65; | H, 4.79; | N, 7.40; | S, 8.47. |
| Found: | C, 66.53; | H, 4.79; | N, 7.53; | S, 8.41. |

### Example 24

### N-(3,4-Dimethyl-5-isoxazolyl)-3'-(2-methylpropyl)-[1,1'-biphenyl]-2-sulfonamide

### A. 3-Bromo-isobutylbenzene

To a solution of 1-iodo-3-bromobenzene (6.0g, 21.2 mmol) in 100 mL of benzene at room temperature under argon, 1.2g (1.06 mmol) of tetrakis(triphenylphosphine)palladium(0) was added and to this mixture a 2M solution in tetrahydrofuran of isobutyl magnesium bromide (10.6 mL) was added dropwise over 15 minutes. The mixture was stirred 2 hours, diluted with 100 mL of water, the organic layer was separated and the aqueous layer was extracted with 2 x 100 mL of ether. The combined organic extracts were dried and evaporated to provide 4.3g of a colorless liquid, which upon distillation in vacuo provided 1.95g (43%) of compound A as a colorless liquid; b.p. 124-125°C (15-20 mm).

### B. 3-isobutyl-phenylboronic acid

Compound B was prepared from compound A as described for compound A of Example 21. Crystallization from ether/hexanes provided compound B as a white solid, m.p. 84-86°C.

### C. N-(3,4-Dimethyl-5-isoxazolyl)-N-[(2-methoxyethoxy)methyl]-3'-(2-Methylpropyl)[1,1' -biphenyl]-2-sulfonamide

Compound C was prepared from Compound B and compound A from Example 4 as described for compound A from Example 12, with refluxing for 6 hours. Chromatography on silica gel using hexanes/ethyl acetate (3:1) afforded compound C as a colorless gum.

### D. N-(3,4-Dimethyl-5-isoxazolyl)-3'-(2-methylpropyl)-[1,1'-biphenyl]-2-sulfonamide

Compound D was prepared from compound C as described for Example 18, with refluxing for 10 hours. Chromatography on silica gel using 3:1 hexanes/ethyl acetate followed by reverse phase preparative high performance liquid chromatography (30x500 mm ODS S10 column using 85% solvent A (90% MeOH, 10% H₂O, 0.1% TFA) and 15% solvent B (10% MeOH, 90% H₂O, 0.1% TFA)) provided Example 24 as a colorless gum.
¹H NMR (CDCl₃): d 1.04 (d, J = 6.4 Hz, 6H), 1.94 (s, 3H), 2.02 (m, 1H), 2.26 (s, 3H), 2.64 (d, J = 7.0 Hz, 2H), 6.66 (br s, 1 H), 7.32-8.16 (m, 8H).

| Analysis for C₂₁H₂₄N₂O₃S-0.42 H₂O | | | | |
|---|---|---|---|---|
| Calc'd: | C, 64.33; | H, 6.39; | N, 7.14; | S, 8.18. |
| Found: | C, 64.31; | H, 6.16; | N, 7.16; | S, 7.99. |

### Example 25

### N-(3,4-Dimethyl-5-isoxazolyl)-4'-(2-methylpropoxy)-[1,1'-biphenyl]-2-sulfonamide

### A. 4-(2-methylpropoxy)-phenylboronic acid

Compound A was prepared from 4-(2-methylpropoxy)bromobenzene and trimethylborate as described for compound A of Example 21. Crystallization from ether/hexanes provided compound A as a white solid.

### B. N-(3,4-Dimethyl-5-isoxazolyl)-N-[(2-methoxyethoxy)methyl]-4'-(2-methylpropoxy)[1,1' -biphenyl]-2-sulfonamide

Compound B was prepared from compound A and compound A from Example 4 as described for compound A from Example 12, with refluxing for 4 hours. Chromatography on silica gel using 40:1 methylene chloride/ethyl acetate afforded compound C as a colorless gum.

### C. N-(3,4-Dimethyl-5-isoxazolyl)-4'-(2-methylpropoxy)-[1,1'-biphenyl]-2-sulfonamide

Compound C was prepared from compound B as described for Example 18, with refluxing for 8 hours. Chromatography on silica gel using 15:1 methylene chloride/ethyl acetate provided Example 25 as a colorless solid, m.p. 50-53°C.

| Analysis for C₂₁H₂₄N₂O₄S-0.7 H₂O | | | | |
|---|---|---|---|---|
| Calc'd: | C, 61.06; | H, 6.20; | N, 6.78; | S, 7.76. |
| Found: | C, 61.28; | H, 5.96; | N, 6.66; | S, 8.11. |

### Example 26

### N-(3,4-Dimethyl-5-isoxazolyl)-4'-(1-methylethoxy)-[1,1'-biphenyl]-2-sulfonamide

### A. 4-(1-methylethoxy)-phenylboronic acid

Compound A was prepared from 4-(1-methylethoxy)bromobenzene and trimethylborate as described for compound A of Example 21. Crystallization from ether/hexanes provided compound A as a white solid.

### B. N-(3,4-Dimethyl-5-isoxazolyl)-N-[(2-methoxyethoxy)methyl]-4'-(1-methylethoxy)[1,1' -biphenyl]-2-sulfonamide.

Compound B was prepared from compound A and compound A from Example 4 as described for compound A from Example 12, with refluxing for 5 hours. Chromatography on silica gel using 4:1 hexanes/ethyl acetate afforded compound B as a colorless gum.

### C. N-(3,4-Dimethyl-5-isoxazolyl)-4'-(1-methylethoxy)-[1,1'-biphenyl]-2-sulfonamide.

Compound C was prepared from compound B as described for Example 18, with refluxing for 2 hours. Chromatography on silica gel using 4:1 hexanes/ethyl acetate provided Example 26 as a colorless solid, m.p. 49-52°C.

| Analysis for C₂₀H₂₂N₂O₄S | | | | |
|---|---|---|---|---|
| Calc'd: | C, 62.16; | H, 5.74; | N, 7.25; | S, 8.30. |
| Found: | C, 61.98; | H, 5.71; | N, 7.12; | S, 8.17. |

### Example 27

### N-(3,4-Dimethyl-5-isoxazolyl)-4'-(phenylmethyloxy)-[1,1'-biphenyl]-2-sulfonamide

### A. 4-Phenylmethyloxy-phenylboronic acid

To a solution of 4-phenylmethyloxy-bromobenzene (6.0g, 23 mmol) in tetrahydrofuran (25 mL) and ether (75 mL) at -78°C under argon, butyllithium (1.6M solution in hexane, 14.25 mL) was added over 15 minutes. The mixture was stirred 15 minutes and transferred via cannula over 15 minutes to a solution of trimethylborate (4.73g, 45.6 mmol) in 50 mL of ether at -78°C under argon. After 30 minutes at -78°C, the solution was warmed to room temperature and stirred for a furthur 60 minutes. 10% aqueous hydrochloric acid was added (150 mL) and after 10 min the solution was extracted with ether (3 x 100 mL). The combined ether extracts were extracted with 1 M sodium hydroxide (3 x 100 mL) and the combined aqueous extracts were acidified with dilute hydrochloric acid to pH 2 and extracted with ether (3 x 100 mL). The combined ether extracts were washed once with water (100 mL), dried and evaporated to afford a white solid which was crystallized from ether/hexanes to provide 1.48g (29%) of pure compound A as a white solid in two crops, m.p. 187-189°C.

### B. N-(3,4-Dimethyl-5-isoxazolyl)-N-[(2-methoxyethoxy)methyl]-4'-(phenylmethyloxy)[1,1' -biphenyl]-2-sulfonamide

Compound B was prepared from compound A and compound A from Example 4 as described for compound A from Example 12. Chromatography on silica gel using 3:1 hexanes/ethyl acetate afforded compound B as a colorless gum.

### C. N-(3,4-Dimethyl-5-isoxazolyl)-4'-(phenylmethyloxy)-[1,1-biphenyl]-2-sulfonamide

Compound C was prepared from compound B as described for Example 18, with refluxing for 18 hours. Chromatography on silica gel using 3:1 hexanes/ethyl acetate followed by reverse phase preparative HPLC (30 x 500 mm ODS S10 column using 85% solvent A (90% MeOH, 10% H₂O, 0.1% TFA) and 15% solvent B (10% MeOH, 90% H₂O, 0.1% TFA)) provided Example 27 as a colorless gum.
¹H NMR (CDCl₃): d 1.93(s, 3H), 2.20 (s, 3H), 5.20 (s, 2H), 6.32 (br s, 1H), 7.14-8.11 (m, 13H).

| Analysis for C₂₄H₂₂N₂O₄S | | | | |
|---|---|---|---|---|
| Calc'd: | C, 66.34; | H, 5.10; | N, 6.45; | S, 7.38. |
| Found: | C, 66.14; | H, 5.00; | N, 6.29; | S, 7.09. |

### Example 28 4'-(1,1-Dimethylethyl)-N-(3,4-dimethyl-5-isoxazolyl)-[1,1'-biphenyl]-2-sulfonamide

### A. 4-t-Butyl-phenylboronic acid

Compound A was prepared from 4-t-butyl-bromobenzene and trimethylborate as described for compound A of Example 21. Crystallization from ether/hexanes provided compound A as white crystals, m.p. 201-203°C.

### B. 4'-(1,1-Dimethylethyl)-N-(3,4-dimethyl-5-isoxazolyl)-N-[(2-methoxyethoxy)methyl][1,1' -biphenyl]-2-sulfonamide

Compound B was prepared from compound A and compound A from Example 4 as described for compound A from Example 12, with refluxing for 4 hours. Chromatography on silica gel using 6:1 hexanes/ethyl acetate afforded compound B as a colorless gum.

### C. 4'-(1,1-Dimethylethyl)-N-(3,4-dimethyl-5-isoxazolyl)-[1,1'-biphenyl]-2-sulfonamide

Compound C was prepared from compound B as described for Example 18, with refluxing for 5 hours. Chromatography on silica gel using 4.5:1 hexanes/ethyl acetate followed by crystallization from ethyl acetate/hexanes provided Example 28 as colorless crystals, m.p. 169-170°C.

| Analysis for C₂₁H₂₄N₂O₃S | | | | |
|---|---|---|---|---|
| Calc'd: | C, 65.60; | H, 6.29; | N, 7.29; | S, 8.34. |
| Found: | C, 65.44; | H, 6.24; | N, 7.26; | S, 8.21. |

### Example 29

### N-(3,4-Dimethyl-5-isoxazolyl)-4'-methoxy-[1,1'-biphenyl]-2-sulfonamide

### A. N-(3,4-Dimethyl-5-isoxazolyl)-4'-methoxy-N-[(2-methoxyethoxy)methyl][1,1'-biphenyl]-2-sulfonamide

Compound A was prepared from 4-methoxybenzeneboronic acid and compound A from Example 4 as described for compound A from Example 12, using toluene rather than benzene and with heating at 95°C for 5 hours. Chromatography on silica gel using 3.5:1 hexanes/ethyl acetate afforded compound A as a colorless gum.

### B. N-(3,4-Dimethyl-5-isoxazolyl)-4'-methoxy-[1,1'-biphenyl]-2-sulfonamide

Compound B was prepared from compound A as described for Example 18, with refluxing for 4 hours. Cooling of the reaction mixture afforded Example 29 as colorless crystals, m.p.179-181°C.

| Analysis for C₁₈H₁₈N₂O₄S | | | | |
|---|---|---|---|---|
| Calc'd: | C, 60.32; | H, 5.06; | N, 7.82; | S, 8.95. |
| Found: | C, 60.14; | H, 5.08; | N, 7.86; | S, 9.24. |

### Example 30

### N-(3,4-Dimethyl-5-isoxazolyl)-4'-[(1-methylethyl)amino][1,1'-biphenyl]-2-sulfonamide

### A. 4'-Amino-N-(3,4-dimethyl-5-isoxazolyl)-N-[(2-methoxyethoxy)methyl][1,1'-biphenyl]-2-sulfonamide

Compound A was prepared from 4-amino-phenylboronic acid and compound A from Example 4 as described for compound A from Example 12, using toluene rather than benzene and with heating at 85°C for 4 hours. Chromatography on silica gel using 1:1 hexanes/ethyl acetate afforded compound A as a colorless gum.

### B. N-(3,4-Dimethyl-5-isoxazolyl)-N'-(methoxyethoxymethyl)-4'-[(1-methylethyl) amino][1,1'-biphenyl]-2-sulfonamide

To compound A (720 mg, 1.67 mmol) and acetone (0.16 mL, 2.17 mmol) in 1,2-dichloroethane (12 mL) at 0°C, acetic acid (0.14 mL) was added over 5 minutes followed by sodium triacetoxyborohydride (460 mg, 2.17 mmol) in portions. The mixture was stirred at room temperature overnight, additional acetone (0.04 ml, 0.54 mmol), acetic acid (0.04 mL) and sodium triacetoxyborohydride (115 mg, 0.54 mmol) were added and the mixture was stirred 40 minutes. The mixture was poured into water (50 mL), ethyl acetate (150 mL) was added and the organic layer was separated, washed with brine, dried and concentrated. The residue was chromatographed on silica gel with 3:1 hexanes/ethyl acetate to afford compund B (590 mg, 75%) as a colorless gum.

### C. N-(3,4-Dimethyl-5-isoxazolyl)-4'-[(1-methylethyl)amino][1,1'-biphenyl]-2-sulfonamide

To a solution of compound B (315 mg, 0.67 mmol) in 95% ethanol (8 mL), 6N aqueous hydrochloric acid (8 mL) was added. The mixture was refluxed for 3 hours and concentrated. Saturated sodium hydrogen carbonate was added until the pH was above 8. The mixture was acidified to ∼pH 5 with acetic acid and extracted with ethyl acetate (3 x 30 mL). The combined organic extracts were washed with brine, dried and concentrated. The residue was chromatographed on silica gel using 2:1hexanes/ethyl acetate to afford Example 30 as a colorless solid (225 mg, 88 %), m.p. 62-64°C.

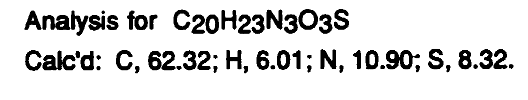

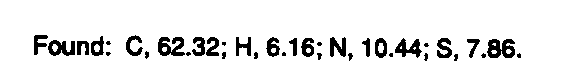

### Example 31

### 2-[[[[2'-[[(3,4-Dimethyl-5-isoxazolyl)amino]sulfonyl]-[1,1'-biphenyl]-4-yl](1-methylethyl)amino]carbonyl] amino]-4-methylpentanoic acid, ethyl ester

### A. 2-[[[[2'-[[(3,4-Dimethyl-5-isoxazolyl)amino]carbonyl]-[1,1'-biphenyl]-4-yl](1 -methylethyl)amino]carbonyl]-amino]-4-methylpentanoic acid, ethyl ester

To a solution of Example 30 (145 mg, 0.38 mmol) in methylene chloride (4.4 mL), ethyl 2-isocyanato-4-methyl valerate (163 mg, 0.88 mmol) was added. The mixture was stirred for two days, diluted with ethyl acetate (25 mL) and washed with water (20 mL) and brine. The organic phase was dried and concentrated and the residue was chromatographed on silica gel using 3:2 hexanes/ethyl acetate to afford Example 31 as a colorless solid (190 mg, 85%), m.p. 58-61°C.

| Analysis for C₂₉H₃₈N₄O₆S | | | | |
|---|---|---|---|---|
| Calc'd: | C, 61.03; | H, 6.71; | N, 9.82; | S, 5.62. |
| Found: | C, 60.59; | H, 6.97; | N, 9.46; | S, 5.29. |

### Example 32

### 2'-Amino-N-(3,4-dimethyl-5-isoxazolyl)-4'-(2-methylpropyl)[1,1'-biphenyl]-2-sulfonamide

### A. 4-isobutyl-2-nitro-phenylboronic acid

To a suspension of 4-isobutyl-phenylboronic acid (0.9 g, 5.05 mmol) in acetic anhydride (9 mL) at -10°C, fuming nitric acid (0.4 mL) was added over 10 minutes. The mixture was stirred for 1 hour, warmed to room temperature and stirred for an additional 1.5 hours. The clear orange solution was added to 100 mL of ice, stirred for 3 hours and azeotroped with water (4 x 100 mL). The residue was partitioned between 25 mL each of ether and water and the ether layer was dried and evaporated to provide 0.75 g of yellow solid. The solid was dissolved in ethyl acetate (25 mL) and the solution was extracted with 1 N·aqueous sodium hydroxide (2 x 25 mL). The combined aqueous extracts were acidified to pH 2 using 2N aqueous hydrochloric acid and extracted with ethyl acetate (2 x 25 mL). The combined organic extracts were washed once with water, dried and evaporated to provide 0.63g of light yellow solid. Reverse phase preparative HPLC on a 30 x 500 mm ODS S10 column using 76% solvent A (90% MeOH, 10% H₂O, 0.1% TFA) and 24% solvent B (10% MeOH, 90% H₂O, 0.1% TFA) provided 0.16 g of compound A as light yellow solid.

### B. 4-Isobutyl-2-amino-phenylboronic Acid

To a suspension of 0.1g of 10% Pd/C in 10 mL of methanol under argon, 0.32g (1.4 mmol) of compound A in 10 mL of methanol was added and the mixture was hydrogenated at 60 psi for 6 hours. The mixture was filtered and the filtrate concentrated to provide 0.3g of compound B as a brown residue.

### C. 2'-Amino-N-(3,4-dimethyl-5-isoxazolyl)-N-[(2-methoxyethoxy)methyl]-4'-(2-methylpropyl)[1,1'-biphenyl]-2-sulfonamide

Compound C was prepared from compound B and compound A from Example 4 as described for compound A from Example 12, with refluxing for 6 hours. Chromatography on silica gel using 3:1 hexanes/ethyl acetate afforded compound C as a colorless gum.

### D. 2'-Amino-N-(3,4-dimethyl-5-isoxazolyl)-4'-(2-methyl-propyl)[1,1'-biphenyl]-2-sulfonamide

Compound D was prepared from compound C as described for Example 12, with refluxing for 3 hours. Preparative reverse phase high performance liquid chromatography (30 x 500 mm ODS S10 column using 60% solvent A (90% MeOH, 10% H₂O, 0.1% TFA) and 40% solvent B (10% MeOH, 90% H₂O, 0.1% TFA)) followed by chromatography on silica gel using 2% methanol in methylene chloride afforded 0.05 g of Example 32, as a light brown foam. m.p. 60-70°C (amorphous).

| Analysis for C₂₁H₂₅N₃O₃S-0.44 H₂O | | | | |
|---|---|---|---|---|
| Calc'd: | C, 61.90; | H, 6.40; | N, 10.31; | S, 7.87. |
| Found: | C, 61.98; | H, 6.23; | N, 10.23; | S, 7.73. |

### Example 33

### N-[2'-[[(3,4-Dimethyl-5-isoxazolyl)amino]sulfonyl][1,1'-biphenyl]-4-yl]]-N-(1-methylethyl)-β -phenylbenzenepropanamide

### A. N-(3,4-Dimethyl-5-isoxazolyl)-N'-(methoxyethoxymethyl)-4'-[(1-methylethyl)-(3,3 -diphenyl-1-oxopropyl)-amino]-[1,1'-biphenyl]-2-sulfonamide

To compound B from Example 30 (60 mg, 0.13 mmol) in methylene chloride (1.3 mL), 3,3-diphenylpropionyl chloride (93 mg, 0.38 mmol) and triethylamine (0.07 mL) were added. The mixture was stirred 2.5 hours, diluted with ethyl acetate (20 mL) and washed with saturated ammonium chloride (2 x 15 mL) and brine, dried and concentrated. The residue was chromatographed on silica gel using 1:1 hexanes/ethyl acetate to afford compound A as a colorless gum (45 mg, 52%).

### B. N-[2'-[[(3,4-Dimethyl-5-isoxazolyl)amino]sulfonyl][1,1'-biphenyl]-4-yl]]-N -(1-methylethyl)-β-phenylbenzenepropanamide

Example 33 was prepared from compound A as described for Example 18, with refluxing for 3 hours. Chromatography on silica gel using 3:1 methylene chloride/ethyl acetate provided Example 33 as a light yellow solid , m.p. 177°C.

| Analysis for C₃₅H₃₅N₃O₄S-0.4 H₂O | | | | |
|---|---|---|---|---|
| Calc'd: | C, 69.96; | H, 6.00; | N, 6.99; | S, 5.34. |
| Found: | C, 70.13; | H, 6.10; | N, 6.82; | S, 5.21. |

### Example 34

### 2'-Nitro-N-(3,4-dimethyl-5-isoxazolyl)-[1,1'-biphenyl]-2-sulfonamide

### A. 2'-Nitro-N-(3,4-dimethyl-5-isoxazolyl)-N-(methoxyethoxymethyl)[1,1'-biphenyl]-2-sulfonamide

Compound A was prepared from 2-nitrophenylboronic acid and compound A from Example 4 as described for compound A from Example 12, using toluene in place of benzene and refluxing for 6 hours. Flash chromatography on silica gel using hexanes/ethyl acetate (2:1) provided compound A as a light yellow gum.

### B. 2'-Nitro-N-(3,4-dimethyl-5-isoxazolyl)-[1,1'-biphenyl]-2-sulfonamide

Example 34 was prepared from compound A as described for Example 12, with refluxing for 4 hours. Flash chromatography on silica gel using hexanes/ethyl acetate (2:1) followed by crystallization from hexanes/ethyl acetate afforded Example 34 as light brown needles, m.p. 128-130°C.

| Analysis calculated for C₁₇H₁₅N₃O₅S | | | | |
|---|---|---|---|---|
| Calc'd: | C, 54.69; | H, 4.05; | N, 11.25; | S, 8.59. |
| Found: | C, 54.67; | H, 3.88; | N, 11.17; | S, 8.59. |

### Example 35

### 5-[[(2-phenyl)phenyl]sulfonyl]amino]-3-methyl-4-isoxazolecarboxylic acid, ethyl ester

### A. [1,1'-biphenyl]-2-sulfonamide

To a degassed solution of 2-bromobenzenesulfonamide (0.7 g, 3.0 mmol) and tetrakis(triphenylphosphine) palladium (0) (0.21 g, 0.18 mmol) in benzene (25 mL) was added 2M aqueous sodium carbonate (15 mL) followed by a solution of phenylboronic acid (0.44 g, 3.6 mmol) in 95% ethanol (25 mL). The yellow two phase solution was refluxed for 18 hours, cooled to room temperature and diluted with water (100 mL). The solution was extracted with ethyl acetate (2 x 100 mL) and the combined organic phases were washed with brine, dried (magnesium sulfate), filtered and evaporated. The residue was chromatographed on silica gel with hexanes/ethyl acetate (2:1) to yield 250 mg (36%) of compound A as a yellow solid.

### B. 5-[[(2-phenyl)phenyl]sulfonyl]amino]-3-methyl-4-isoxazolecarboxylic acid, ethyl ester

A solution of compound A (187 mg, 0.82 mmol), 3-methyl-4-ethoxycarbonyl-5-bromoisoxazole (197 mg, 0.42 mmol) and cesium carbonate (274 mg, 0.42 mmol) in dry dimethylformamide (4 mL) was heated at 55°C for 18 hours. The solution was cooled to room temperature, diluted with water (40 mL) and acidified to pH 4 with 6N aqueous hydrogen chloride. The tan precipitate was collected by filtration, rinsed with water, and dried to afford 110 mg (36 %) of Example 35 as a tan solid, m.p. 126-128°C.

| Analysis calculated for C₁₉H₁₈N₂O₅S - 0.35 H₂O | | | | |
|---|---|---|---|---|
| Calc'd: | C, 58.10; | H, 4.80; | N, 7.13; | S, 8.16. |
| Found: | C, 58.19; | H, 4.59; | N, 7.04; | S, 8.06. |

### Example 36

### N-(3-Methyl-4-phenylmethyl-5-isoxazolyl)-4'-(2-methylpropyl)-(1,1'-biphenyl]-2-sulfonamide

### A. N-(2-Bromobenzenesulfonyl)-pyrrole

Potassium hydride (35% oil dispersion, 5.76 g, 50 mmol; washed three times with hexanes) was covered with dry tetrahydrofuran (200 mL) and the suspension was cooled to 0°C. Pyrrole (passed through activity I basic alumina, 4.16 ml, 60 mmol) in tetrahydrofuran (60 mL) was added dropwise over 20 min. The ice bath was removed and the solution was allowed to stir at ambient temperature until the gas evolution ceased (20 minutes) whereupon 2-bromobenzenesulfonyl chloride (10.22 g, 40 mmol) in tetrahydrofuran (60 mL) was added dropwise over 20 minutes. After stirring for 1 hour, the mixture was filtered through Celite AFA and the filter pad was rinsed with tetrahydrofuran (100 mL). The filtrate was evaporated and the resulting white solid was recrystallized from methanol to afford 7.47 g (65%) of compound A, mp 85.0-87.0°C.

### B. N-(4'-(2-Methylpropyl)-1,1'-biphenylsulfonyl)pyrrole

Compound B was prepared from compound A and compound A from Example 21 as described for compound A from example 12, using toluene rather than benzene and with heating at 80°C for 2 hours. Chromatography on silica gel using 1:1 hexanes/methylene chloride afforded compound B as an oil.

### C. 4'-(2-Methylpropyl)-1,1'-biphenyl-2-sulfonic acid, sodium salt

A solution of compound B and 5N sodium hydroxide (53 mL) in methanol (70 mL) was refluxed for 6.5 hours. Evaporation of the methanol afforded a white solid which was collected and dried under vacuum. Recrystallization from water (40 mL) afforded 3.05 g (88%) of compound C as a white solid.

### D. 4'-(2-Methylpropyl)-1,1'-biphenyl-2-sulfonylchloride

Compound C (1.6 g, 5 mmol) and phosphorus pentachloride (3.1 g, 15 mmol) were ground together with a glass rod and the mixture was heated at 60°C for 2.5 hours. Ice water was added and the mixture was extracted twice with ethyl acetate. The combined organic layers were washed with brine, dried (magnesium sulfate) and evaporated to afford 1.45 g (94%) of compound D.

### E. N-(3-Methyl-4-phenylmethyl-5-isoxazolyl)-4'-(2-methylpropyl)-[1,1'-biphenyl]-2-sulfonamide

Compound D (0.15 g, 0.48 mmol) in pyridine (0.1 mL) was added to a solution of 3-methyl-4-phenylmethyl-5-isoxazolamine (0.12 g, 0.64 mmol) and dimethylaminopyridine (13 mg, 0.1 mmol) in pyridine (0.2 mL). The solution was stirred at 75°C for 2.5 hours, cooled to room temperature and diluted with water. The solution was adjusted to pH 3 with 1N hydrochloric acid and extracted with ether (2 x 50 ml). The combined organic layers were washed with brine, dried (magnesium sulfate) and evaporated. The residue was subjected to flash chromatography (silica, 7% ethyl acetate/methylene chloride) and the partially purified material was subjected to flash chromatography (silica, ether) to afford 30 mg (15%) of pure Example 36 as an oil which solidified upon standing, mp 137.0-138.5°C:

| Analysis calculated for C₂₇H₂₈N₂O₃S-0.86 H₂O | | | | |
|---|---|---|---|---|
| Calc'd: | C, 68.12; | H, 6.29; | N, 5.88; | S, 6.73. |
| Found: | C, 68.38; | H, 6.04; | N, 6.23; | S, 6.31. |

### Example 37

### N-(4,5-Dimethyl-3-isoxazolyl)-4'-(2-methylpropyl)-[1,1'-biphenyl]-2-sulfonamide

Compound D of Example 36 (0.56 g, 1.8 mmol) in pyridine (0.8 mL) was added to a solution of 4,5-dimethyl-3-isoxazolamine (0.25 g, 2.2 mmol) and 4-dimethylaminopyridine (44 mg, 0.4 mmol) in pyridine (0.7 mL). The solution was stirred at 75°C for 2.5 hours, cooled to room temperature and diluted with water (10 mL). The solution was adjusted to pH 3 with 6N hydrochloric acid and extracted with ether (2 x 80 mL). The combined organic layers were washed with brine, dried (magnesium sulfate) and evaporated. Flash chromatography (silica, 20% ethyl acetate/hexanes) afforded 0.33 g (48%) of Example 37. Recrystallization from ether/hexane afforded an analytical sample as a white crystalline solid, mp 131.5-133.0°C.

| Analysis calculated for C₂₁H₂₄N₂O₃S | | | | |
|---|---|---|---|---|
| Calc'd: | C, 65.60; | H, 6.29; | N, 7.29; | S, 8.34. |
| Found: | C, 65.64; | H, 6.33; | N, 7.32; | S, 8.31. |

### Example 38

### 4'-(2-Methylpropyl)-2'-Methoxy-N-(3,4-dimethyl-5-isoxazolyl)-[1,1'-biphenyl]-2-sulfonamide

### A. 4-isobutyl-2-methoxy-phenylboronic acid

To a solution of 4-(2-methylpropyl)-2-methoxybenzene (4.0 g, 24 mmol) in ether (100 mL) under argon at -78°C, tetramethylethylenediamine (11 mL, 73 mmol) was added followed by t-butyllithium (1.7 M solution in pentane, 43 mL) added over 5 minutes. The mixture was warmed to room temperaure, stirred for 5 hours, cooled to -78°C and trimethylborate (7.6 g) was added in one portion. The solution was warmed to room temperature, stirred ovemight, cooled to 0°C and 20% aqueous hydrochloric acid (250 mL) was added. The solution was extracted with ether and the combined ether extracts were extracted three times with 1 M sodium hydroxide. The precipitate which formed was collected and added to the combined aqueous extracts. This mixture was acidified with dilute hydrochloric acid to pH 2 and the solution was extracted twice with ether. The combined ether extracts were washed once with water, dried and evaporated. The white solid was crystallized from hexanes in two crops to provide 2.1g (42%) of compound A as a white solid, m.p. 68-75°C.

### B. 4'-(2-Methylpropyl)-2'-Methoxy-N-(3,4-dimethyl-5-isoxazolyl)-N-(methoxyethoxymethyl)[1,1'-biphenyl]-2-sulfonamide

Compound B was prepared from compound A and compound A from Example 4 as described for compound A from Example 12, using toluene in place of benzene and refluxing for 6 hours. Flash chromatography on silica gel using hexanes/ethyl acetate (3:1) provided compound B as a colorless gum.

### C. 4'-(2-Methylpropyl)-2'-Methoxy-N-(3,4-dimethyl-5-isoxazolyl)-[1,1'-biphenyl]-2-sulfonamide

Example 38 was prepared from compound B as described for Example 12, using 6N hydrochloric acid and with refluxing for 3 hours. Crystallization from hexanes/ethyl acetate afforded Example 38 as a white crystalline solid, m.p. 143-144°C.

| Analysis calculated for C₂₂H₂₆N₂O₄S-0.38 H₂O | | | | |
|---|---|---|---|---|
| Calc'd: | C, 62.71; | H, 6.40; | N, 6.65; | S, 7.61. |
| Found: | C, 62.77; | H, 6.35; | N, 6.59; | S, 7.85. |

### Example 39

### 4'-(2-Methylpropyl)-2'-hydroxy-N-(3,4-dimethyl-5-isoxazolyl)-[1,1'-biphenyl]-2-sulfonamide

To a solution of Example 38 (0.3 g, 0.72 mmol) in dry methylene chloride (25 mL) at -78°C under argon, boron tribromide (1.1 mL of a 1M solution in methylene chloride) was added. The solution was stirred for 3 hours at -78°C and was stirred overnight as it warmed to room temperature. The solution was diluted with methylene chloride, washed twice with water, dried and evaporated. The residue was chromatographed on silica gel using 1% methanol/methylene chloride and the white foamy solid was crystallized from hexanes/ethyl acetate to afford 0.1g of Example 39 as colorless prisms, m.p. 175°C.

| Analysis calculated for C₂₁H₂₄N₂O₄S-0.46 H₂O | | | | |
|---|---|---|---|---|
| Calc'd: | C, 61.70; | H, 6.14; | N, 6.85; | S, 7.84. |
| Found: | C, 61.70; | H, 6.12; | N, 6.66; | S, 7.99. |

### Example 40

### N-(3-Methyl-4-nitro-5-isoxazolyl)-4'-(2-methylpropyl)[1,1'-biphenyl]-2-sulfonamide

Example 40 was prepared from compound D of Example 36 and 3-methyl-4-nitro-5-isoxazolamine as described for Example 37, with stirring at room temperature for 5 days. After flash chromatography (silica, 50% ethyl acetate/methylene chloride), the crude product was partitioned between ethyl acetate and water taken to pH 1 with 6 N hydrochloric acid. The aqueous phase was extracted twice with ethyl acetate and the combined organic phases were washed with brine, dried (magnesium sulfate) and evaporated. Recrystallization from methylene chloride/hexanes afforded Example 40 as a yellow crystalline solid, mp 124-126°C.

| Analysis calculated for C₂₀H₂₁N₃O₅S | | | | |
|---|---|---|---|---|
| Calc'd: | C, 57.82; | H, 5.09; | N, 10.11; | S, 7.72. |
| Found: | C, 57.89; | H, 5.12; | N, 10.25; | S, 7.72. |

### Example 41

### N-(4-Methyl-5-isoxazolyl)-4'-(2-methylpropyl)-[1,1'-biphenyl]-2-sulfonamide

Example 41 was prepared from compound D of Example 36 and 4-methyl-5-isoxazolamine as described for Example 37, with stirring at room temperature for 2.2 hours. Flash chromatography (silica, 10% ethyl acetate/methylene chloride) followed by trituration with ether afforded Example 41 as a white crystalline solid, mp 153.0-155.5°C.

| Analysis calculated for C₂₀H₂₂N₂O₃S-0.39 H₂O | | | | |
|---|---|---|---|---|
| Calc'd: | C, 63.65; | H, 6.08; | N, 7.42; | S, 8.49. |
| Found: | C, 63.39; | H, 5.90; | N, 7.68; | S, 8.40. |

### Example 42

### 4'-(2-Methylpropyl)-N-(4,5,6,7-tetrahydro-2,1-benzisoxazol-3-yl)-[1,1'-biphenyl]-2-sulfonamide

Example 42 was prepared from compound D of Example 36 and 4,5,6,7-tetrahydro-2,1-benzisoxazol-3-amine as described for Example 37, with stirring at 75°C for 2 hours. Flash chromatography (silica, 30% ethyl acetate/methylene chloride) followed by a second flash chromatography (silica, ether) followed by recrystallization from methylene chloride/hexanes afforded Example 42 as an off-white crystalline solid, mp 111.0-114.5°C.

| Analysis calculated for C₂₃H₂₆N₂O₃S | | | | |
|---|---|---|---|---|
| Calc'd: | C, 67.29; | H, 6.38; | N, 6.82; | S, 7.81. |
| Found: | C, 66.93; | H, 6.36; | N, 7.04; | S, 7.57. |

### Example 43

### 4-Amino-4'-(2-methylpropyl)-N-(3,4-dimethyl-5-isoxazolyl)-[1,1'-biphenyl]-2-sulfonamide

### A. 2-Bromo-4-(1-oxoethylamino)-benzenesulfonyl chloride

To 2-bromo-4-(1-oxoethylamino)-benzene (6.2 g, 29 mmol) was added chlorosulfonic acid (20 mL). The solution was heated at 57°C for 3 hours, an additional 10 mL of chlorosulfonic acid was added and the solution was heated at 67°C for 6 hours. The mixture was added dropwise to ice water and the heterogeneous mixture was extracted with ethyl acetate. The organic extract was washed once with brine, dried (magnesium sulfate) and evaporated. The residue was dissolved in ethyl acetate (50 mL) and the solution was filtered. The insoluble solid was rinsed twice with ethyl acetate and the combined filtrates were evaporated to afford 6.9 g (82%) of crude compound A as a brown foamy gum.

### B. 2-Bromo-4-(1-oxoethylamino)-N-(3,4-dimethyl-5-isoxazolyl)-benzenesulfonamide

A solution of crude compound A (6.9 g, 22 mmol), 3,4-dimethyl-5-isoxazolamine (3.96 g, 35.3 mmol) and dimethylaminopyridine (0.42 g, 3.5 mmol) in pyridine (25 mL) was heated at 78°C for 3.5 hours. The solvent was evaporated and the residue was partitioned between ethyl acetate and aqueous potassium hydrogen sulfate. The aqueous layer was washed with ethyl acetate and the combined organic layers were washed with brine, dried (magnesium sulfate) and evaporated. The brown solid was subjected to flash chromatography on silica with 90% ethyl acetate/hexanes to afford 3.72 g (43%) of crude compound B as a yellow foam.

### C. 2-Bromo-4-(1-oxoethylamino)-N-(methoxyethoxymethyl)-N-(3,4-dimethyl-5 -isoxazolyl)-benzenesulfonamide

To sodium hydride (0.32 g of an 80% oil dispersion, washed three times with hexanes; 10.5 mmol) was added dropwise compound A (3.72 g, 9.6 mmol) in dry tetrahydrofuran (75 mL). The resulting supension was cooled to 0°C and methoxyethoxymethylchloride (1.09 mL, 9.6 mmol) in dry tetrahydrofuran (10 mL) was added dropwise. The solution was allowed to warm to room temperature overnight, ethyl acetate was added and the solution was extracted with aqueous sodium hydrogen carbonate and brine, dried (magnesium sulfate) and evaporated to afford 3.82 g of a yellow foamy gum. Flash chromatography on silica with 80% ethyl acetate/hexanes afforded 0.52 g (11%) of clean compound C as a yellow foamy gum as well as 0.92 g of less pure material.

### D. 4'-(2-Methylpropyl)-4-(1-oxoethylamino)-N-(methoxyethoxymethyl)-N-(3,4-dimethyl-5 -isoxazolyl)-[1,1'-biphenyl]-2-sulfonamide

Compound D was prepared from compound C and 4-(2-methylpropyl)-benzeneboronic acid as described for compound A of Example 12, using toluene rather than benzene, with heating at 84°C for 90 minutes. Flash chromatography on silica gel using hexanes/ethyl acetate (1:1) afforded compound D as a colorless gum.

### E. 4-Amino-4'-(2-methylpropyl)-N-(3,4-dimethyl-5-isoxazolyl)-[1,1'-biphenyl]-2-sulfonamide

A solution of compound D (0.40 g, 0.75 mmol) in a 1:1 mixture of 6N hydrochloric acid:95% ethanol (30 mL) was heated at reflux for 4.5 hours. The ethanol was evaporated and the aqueous solution was taken to pH 3.5 with aqueous sodium hydrogen carbonate and extracted three times with ethyl acetate. The combined organic phases were washed with brine, dried (magnesium sulfate) and evaporated. Flash chromatography on silica with 25%, then 33% ethyl acetate/hexanes afforded 40 mg of Example 43 as a white foamy solid, m.p. 69-79°C.

| Analysis calculated for C₂₁H₂₅N₃O₃S-0.18 H₂O | | | | |
|---|---|---|---|---|
| Calc'd: | C, 62.63; | H, 6.35; | N, 10.43; | S, 7.96. |
| Found: | C, 63.03; | H, 6.62; | N, 10.03; | S, 7.55. |

### Example 44

### 2'-Fluoro-4'-(2-methylpropyl)-N-(3,4-dimethyl-5-isoxazolyl)-[1,1'-biphenyl]-2-sulfonamide

### A. 3-Fluoro-(2-methyl-1-propenyl)-benzene

To a solution of isopropyl triphenylphosphonium iodide (32 g, 74 mmol) in ether (620 mL) at -15°C, n-butyllithium (1.6 M in hexane, 55 mL, 88 mmol) was added dropwise. The mixture was stirred at room temperature for 3 hours, cooled to -78°C, and 3-fluorobenzaldehyde (10.1 g, 81 mmol) was added slowly. The mixture was stirred at room temperature overnight, cold water was added and the mixture was stirred for several minutes and filtered. The organic phase of the filtrate was separated and washed three times with water, dried (magnesium sulfate), filtered and concentrated. The residue was chromatographed on silica gel with pentane to afford 10 g of compound A (90%) as a colorless liquid.

### B. 3-Fluoro-(2-methylpropyl)-benzene

A mixture of compound A (8 g, 53 mmol) and 10% palladium on carbon (1.2g) in ethyl acetate (80 mL) was hydrogenated at 60 psi for 1 hour. The mixture was filtered, the filtrate was concentrated and the residue was distilled under vacuum to afford compound B as a colorless liquid (5.9 g, 74%), b.p. 113°C/150 mmHg.

### C. 2-Fluoro-4-(2-methylpropyl)-phenylboronic acid

Compound C was prepared from compound B as described for compound A of Example 38, with the following changes. The t-butyllithium solution was stirred at - 78°C for 5 hours. Initial extractions were performed with methylene chloride and the combined organic phases were concentrated to 100 mL before base extraction, during which no precipitate was observed. Trituration with hexanes afforded compound C as a white solid, m.p. 96-100°C.

### D. 2'-Fluoro-4'-(2-methylpropyl)-N-(methoxyethoxymethyl)-N-(3,4-dimethyl-5 -isoxazolyl)-[1,1'-biphenyl]-2-sulfonamide

Compound D was prepared from compound C and compound A from Example 4 as described for compound A of Example 12, using toluene rather than benzene, with heating at 80°C for 3 hours. Flash chromatography on silica gel using hexanes/ethyl acetate (4:1) afforded compound D as a colorless gum.

### E. 2'-Fluoro-4'-(2-methylpropyl)-N-(3,4-dimethyl-5-isoxazolyl)-[1,1'-biphenyl]-2-sulfonamide

Example 44 was prepared from compound D as described for Example 12, using 6N hydrochloric acid and with refluxing for 3 hours. Flash chromatography on silica gel using hexanes/ethyl acetate (3:1) followed by crystallization from hexanes/ethyl acetate afforded Example 44 as colorless crystals, m.p. 139-141°C.

| Analysis calculated for C₂₁H₂₃N₂O₃FS. | | | | | |
|---|---|---|---|---|---|
| Calc'd: | C, 62.67; | H, 5.76; | F, 4.72; | N, 6.96; | S, 7.97. |
| Found: | C, 62.81; | H, 5.83; | F, 4.59; | N, 6.97; | S, 8.03. |

### Example 45

### 4-Methoxy-4'-(2-methylpropyl)-N-(3,4-dimethyl-5-isoxazolyl)-[1,1'-biphenyl]-2-sulfonamide

### A. N-(3,4-dimethyl-5-isoxazolyl)-2-bromo-4-methoxybenzenesulfonamide

To chlorosulfonic acid (10 mL) at 0 °C was added dropwise 3-bromoanisole (9.3 g, 50 mmol) at such a rate that the internal temperature remained below 5 °C. The mixture was stirred at 0 °C for 2 hours and added dropwise to crushed ice. The mixture was extracted three times with methylene chloride and the combined organic layers were dried (sodium sulfate) and concentrated to afford a mixture of 2-bromo-4-methoxy-benzenesulfonyl chloride and 4-bromo-2-methoxy-benzenesulfonyl chloride (756 mg, 5%) as a colorless oil. A solution of this material (756 mg, 1 mmol), 3,43,4-dimethyl-5-isoxazolamine (386 mg, 3.44 mmol) and 4-dimethylaminopyridine (65 mg, 0.53 mmol) in dry pyridine (5 mL) was heated at 70 °C for 2 hours. The mixture was cooled to room temperature and was poured into water . The pH of the mixture was adjusted to 8 with saturated sodium bicarbonate solution and the mixture was extracted twice with ether. The aqueous layer was brought to pH 2 with 6 N hydrochloric acid and was extracted three times with ether. These organic extracts were combined, dried (sodium sulfate) and concentrated to provide a mixture of compound A and the regiomeric N-(3,4-dimethyl-5-isoxazolyl)-4-bromo-2-methoxy-benzenesulfonamide as a tan foam (743 mg). Chromatography (flash, silica, 2% methanol/chloroform) provided pure compound A (288 mg, 30%).

### B. 2-Bromo-4-methoxy-N-(methoxyethoxymethyl)-N-(3,4-dimethyl-5-isoxazolyl)-benzenesulfonamide

To a 0 °C suspension of sodium hydride (60% oil dispersion, 33.5 mg, 0.837 mmol) in dry tetrahydrofuran (2 mL) was added dropwise a solution of compound A (288 mg, 0.797 mmol) in dry tetrahydrofuran (4 mL). After stirring at 0°C for 30 min, methoxyethoxymethyl chloride (0.100 mL, 0.877 mmol) was added dropwise. After 2 hours, an additional portion of methoxyethoxymethyl chloride (0.015 mL) was added. After an additional 1 hour, the mixture was poured into saturated sodium chloride and 1 N hydrochloric acid was added. The mixture was extracted three times with ethyl acetate and the combined organic layers were dried (magnesium sulfate) and concentrated. Chromatography (flash, silica, 30% ethyl acetate/hexanes) provided compound B as a transparent oil (293 mg, 88%).

### C. 4-Methoxy-4'-(2-methylpropyl)-N-(methoxyethoxymethyl)-N-(3,4-dimethyl-5 -isoxazolyl)-[1,1'-biphenyl]-2-sulfonamide

Compound C was prepared from compound B and 4-(2-methylpropyl)-benzeneboronic acid as described for compound A of Example 12, using toluene rather than benzene, with heating at reflux for 30 minutes. Flash chromatography on silica gel using hexanes/ethyl acetate (4:1) afforded compound D as a colorless gum. Chromatography (flash, silica, 30% ethyl acetate/hexanes) yielded compound C as a colorless oil (292 mg, 81%).

### D. 4-Methoxy-4'-(2-methylpropyl)-N-(3,4-dimethyl-5-isoxazolyl)-(1,1'-biphenyl]-2-sulfonamide

Example 45 was prepared from compound C as described for Example 12, using 6N hydrochloric acid and with heating at 80°C for 19 hours. Flash chromatography on silica gel using hexanes/ethyl acetate (2:1) afforded Example 45 as a colorless glassy solid.

| Analysis calculated for C₂₂H₂₆N₂O₄S-0.11 H₂O. | | | | |
|---|---|---|---|---|
| Calc'd: | C, 63.44; | H, 6.35; | N, 6.76; | S, 7.97. |
| Found: | C, 63.52; | H, 6.34; | N, 6.65; | S, 8.03. |

### Example 46

### 2'-Amino-N-(3,4-dimethyl-5-isoxazolyl)-4'-(2-methylpropyl)[1,1'-biphenyl]-2-sulfonamide

### A. 3-(2-Methyl-1-propenyl)-nitrobenzene

To isopropyl triphenylphosphonium iodide (74 g, 170 mmol) in 1:1 ether:tetrahydrofuran (850 mL) at -15°C, n-butyllithium (1.6 M in hexane, 118 mL, 188 mmol) was added dropwise. The mixture was stirred at room temperature for 3 hours, cooled to -50°C, and a solution of 3-nitrobenzaldehyde (28.4 g, 188 mmol) in tetrahydrofuran (60 mL) was added slowly. The mixture was stirred at room temperature overnight, cold water and hexane were added and the mixture was stirred for several minutes and filtered. The organic phase of the filtrate was separated and washed three times with water, dried (magnesium sulfate) and concentrated. The residue was chromatographed on silica gel with 50:1 hexanes/ethyl acetate to afford compound A (23 g, 76%) as a light yellow liquid.

### B. 3-(2-Methyl-1-propenyl)-aniline

A mixture of compound A (4.0 g, 22 mmol) and 5% Pt/C (400 mg) in methanol (40 mL) was hydrogenated at 45 psi overnight. The mixture was filtered and the filtrate concentrated to provide compound B (3.11 g, 92%).

### C. N-(2,2-Dimethyl-1-oxopropyl)-3-(2-methyl-1-propenyl)-aniline

To compound B (3.11 g, 21.1 mmol) and trimethylacetyl chloride (3.31 g, 27.5 mmol) in methylene chloride (53 mL) at 0°C, triethylamine (4.28 g, 42.2 mmol) was added slowly. The mixture was stirred at 0°C for 1 hour and at room temperature for 15 minutes and poured into ice water. The aqueous layer was extracted twice with ethyl acetate and the combined organic phases were washed with brine, dried and concentrated. The residue was chromatographed on silica gel with 25:2 hexanes/ethyl acetate to provide compound C (3.81g, 78%) as a white solid.

### D. N-(2,2-Dlmethyl-1-oxopropyl)-3-(2-methyl-1-propyl)-aniline

A mixture of compound C (3.47 g, 15 mmol) and 10% Pd/C (520 mg) in ethyl acetate (35 mL) was hydrogenated at 60 psi for 1 hour. The mixture was filtered and the filtrate concentrated to provide compound D (3.41 g, 98%).

### E. 2-(2,2-Dimethyl-1-oxo-1-propylamino)-4-(2-methyl-1-propyl)-phenylboronic acid

To compound D (2.86 g,12.3 mmol) and tetramethylethylenediamine (4.28 g, 36.8 mmol) in ether (25 mL) at - 40°C, t-butyllithium (1.7 M in pentane, 21.6 mL, 36.8 mmol) was added dropwise. The solution was stirred at room temperature for 2.5 hours, cooled to -20°C and trimethylborate (3.82 g, 36.8 mmol) was added slowly. The mixture was stirred at -10°C to 0°C for 1 hour and at room temperature for 3 hours, cooled to 0°C and 10% aqueous hydrochloric acid was added. The aqueous layer was extracted three times with methylene chloride and the combined organic phases were washed with brine, dried and concentrated. The residue was triturated with ether to afford compound E as a white solid (2.52 g, 74%), m.p.>250°C.

### F. 2'-(2,2-Dimethyl-1-oxo-1-propylamino)-N-(3,4-dimethyl-5-isoxazolyl)-N-[(2 -methoxyethoxy)methyl]-4'-(2-methyl-propyl)[1,1'-biphenyl]-2-sulfonamide

Compound F was prepared from compound E and compound A from Example 4 as described for compound A from Example 12, using toluene in place of benzene and heating at 75°C for 7 hours. Flash chromatography on silica gel using hexanes/ethyl acetate (4:1) provided compound F as a colorless gum.

### G. 2'-Amino-N-(3,4-dlmethyl-5-isoxazolyl)-4'-(2-methyl-propyl)[1,1'-biphenyl]-2-sulfonamide

To a solution of compound F (354 mg, 0.62 mmol) in 95% ethanol (20 mL), 50% sulfuric acid (20 mL) was added and the mixture was heated at reflux for 3.5 hours. The mixture was cooled and poured onto iced 30% ammonium hydroxide. The mixture was acidified to pH<5 with acetic acid and extracted four times with ethyl acetate. The combined organic extracts were washed with brine, dried and concentrated. Preparative HPLC (30 x 500 mm ODS S10 column using 65% solvent A (90% methanol, 10% water, 0.1% trifluoroacetic add) and 35% solvent B (10% methanol, 90% water, 0.1%trifluoroacetic acid)) provided a solid which was further purified by chromatography on silica using 2% methanol/methylene chloride to afford Example 46 as a white solid, m.p. 60-70°C (amorphous).

| Analysis calculated for C₂₁H₂₅N₃O₃S-0.25 H₂O. | | | | |
|---|---|---|---|---|
| Calc'd: | C, 62.44; | H, 6.36; | N, 10.40; | S, 7.94. |
| Found: | C, 62.65; | H, 6.23; | N, 10.19; | S, 7.67. |

## Claims

1. A compound of the formula 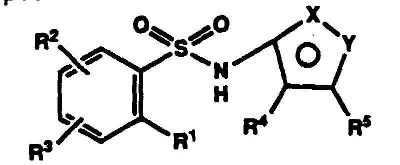 wherein:
one of X and Y is N and the other is O;
R¹, R² and R³ are each independently
(a) hydrogen, except that R¹ is other than hydrogen;
(b) alkyl, alkenyl, alkynyl, alkoxy, cycloalkyl, cycloalkylalkyl, cycloalkenyl, cycloalkenylalkyl, aryl, aryloxy, aralkyl, or aralkoxy, any of which may be substituted with Z¹, Z² and Z³;
(c) halo;
(d) hydroxyl;
(e) cyano;
(f) nitro;
(g) -C(O)H or -C(O)R⁶;
(h) -CO₂H or -CO₂R⁶;
(i) -SH, -S(O)ₙR⁶, -S(O)ₘ-OH, -S(O)ₘ-OR⁶, -O-S(O)ₘ-R⁶, -O-S(O)ₘOH, or -O-S(O)ₘ-OR⁶;
(j) -Z⁴-NR⁷R⁸; or
(k) -Z⁴-N(R¹¹)-Z⁵-NR⁹R¹⁰;
R⁴ and R⁵ are each independently
(a) hydrogen;
(b) alkyl, alkenyl, alkynyl, alkoxy, cycloalkyl, cycloalkylalkyl, cycloalkenyl, cycloalkenylalkyl, aryl, aryloxy, aralkyl, or aralkoxy, any of which may be substituted with Z¹, Z² and Z³;
(c) halo;
(d) hydroxyl;
(e) cyano;
(f) nitro;
(g) -C(O)H or -C(O)R⁶;
(h) -CO₂H or -CO₂R⁶;
(i) -SH, -S(O)ₙR⁶, -S(O)ₘ-OH, -S(O)ₘ-OR⁶, -O-S(O)ₘ-R⁶, -O-S(O)ₘOH, or -O-S(O)ₘ-OR⁶;
(j) -Z⁴-NR⁷R⁸;
(k) -Z⁴-N(R¹¹)-Z⁵-NR⁹R¹⁰; or
(l) R⁴ and R⁵ together are alkylene or alkenylene (either of which may be substituted with Z¹, Z² and Z³), completing a 4- to 8-membered saturated, unsaturated or aromatic ring together with the carbon atoms to which they are attached;
R⁶ is alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
R⁷ is
(a) hydrogen;
(b) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
(c) cyano;
(d) hydroxyl;
(e) -C(O)H or -C(O)R⁶;
(f) -CO₂R⁶; or
(g) -SH, -S(O)ₙR⁶, -S(O)ₘ-OH, -S(O)ₘ-OR⁶,
-O-S(O)ₘ-R⁶, -O-S(O)ₘOH, or -O-S(O)ₘ-OR⁶, except when Z⁴ is -S(O)ₙ-;
R⁸ is
(a) hydrogen;
(b) -C(O)H or -C(O)R⁶, except when Z⁴ is -C(O)- and R⁷ is -C(O)H, -C(O)R⁶ or -CO₂R⁶; or
(c) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³; or
R⁷ and R⁸ together are alkylene or alkenylene (either of which may be substituted with Z¹, Z² and Z³), completing a 3- to 8-membered saturated, unsaturated or aromatic ring together with the nitrogen atom to which they are attached;
R⁹ is
(a) hydrogen;
(b) hydroxyl;
(c) -C(O)H or -C(O)R⁶;
(d) -CO₂R⁶;
(e) -SH, -S(O)ₙR⁶, -S(O)ₘ-OH, -S(O)ₘ-OR⁶, -O- S(O)ₘ-R⁶, -O-S(O)ₘOH or -O-S(O)ₘ-OR⁶; or
(f) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
R¹⁰ is
(a) hydrogen;
(b) -C(O)H or -C(O)R⁶, except when Z⁵ is -C(O)- and R⁹ is -C(O)H, -C(O)R⁶ or -CO₂R⁶; or
(c) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
R¹¹ is
(a) hydrogen;
(b) hydroxyl;
(c) -C(O)H, -C(O)R⁶ or CO₂R⁶; or
(d) alkyl, alkenyl, alkynyl, alkoxy, cycloalkyl, cycloalkylalkyl, cycloalkenyl, cydoalkenylalkyl, aryl, or aralkyl, any of which may be substituted with Z¹, Z² and Z³;
or any two of R⁹, R¹⁰ and R¹¹ together are alkylene or alkenylene (either of which may be substituted with Z¹, Z² and Z³), completing a 3- to 8-membered saturated, unsaturated or aromatic ring together with the atoms to which they are attached;
Z¹, Z² and Z³ are each independently
(a) hydrogen;
(b) halo;
(c) hydroxy;
(d) alkyl;
(e) alkenyl;
(f) aralkyl;
(g) alkoxy;
(h) aryloxy
(i) aralkyloxy
(j) -SH, -S(O)ₙZ⁶, -S(O)ₘ-OH, -S(O)ₘ-OZ⁶, -O- S(O)ₘ-Z⁶, -O-S(O)ₘOH, or -O-S(O)ₘ-OZ⁶;
(k) oxo;
(l) nitro;
(m) cyano;
(n) -C(O)H or-C(O)Z⁶;
(o) -CO₂H or -CO₂Z⁶;
(p) -Z⁴-NZ⁷Z⁸;
(q) -Z⁴-N(NR¹¹)-Z⁵-Z⁶; or
(r) -Z⁴-N(Z¹¹)-Z⁵-NZ⁷Z⁸;
Z⁴ and Z⁵ are each independently
(a) a single bond;
(b) -Z⁹-S(O)ₙZ¹⁰-;
(c) -Z⁹-C(O)-Z¹⁰-;
(d) -Z⁹-C(S)-Z¹⁰-;
(e) -Z⁹-O-Z¹⁰-;
(f) -Z⁹-S-Z¹⁰-; or
(g) -Z⁹-O-C(O)-Z¹⁰-;
Z⁶, Z⁷ and Z⁸ are each independently hydrogen, alkyl, alkenyl, alkynyl, cycloalkyl, cycloalkylalkyl, cycloalkenyl, cycloalkenylalkyl, aryl, or aralkyl, or Z⁷ and Z⁸ together are alkylene or alkenylene, completing a 3- to 8-membered saturated, unsaturated or aromatic ring together with the nitrogen atom to which they are attached;
Z⁹ and Z¹⁰ are each independently a single bond, alkylene, alkenylene, or alkynylene;
Z¹¹ is
(a) hydrogen;
(b) hydroxyl;
(c) -C(O)H, -C(O)Z⁶ or CO₂Z⁶; or
(d) alkyl, alkenyl, alkynyl, alkoxy, cycloalkyl, cycloalkylalkyl, cycloalkenyl, cycloalkenylalkyl, aryl, or aralkyl;
or any two of Z⁷, Z⁸ and Z¹¹ together are alkylene or alkenylene, completing a 3- to 8-membered saturated, unsaturated, or aromatic ring together with the atoms to which they are attached;
m is 1 or 2; and
n is 0,1, or 2;
with the proviso that such compound is not 4-amino-2-fluoro-N-(5-methyl-3-isoxazolyl)benzenesulfonamide, 2-amino-N-(5-methyl-3-isoxazolyl)-4-(trifluoromethyl)benzenesulfonamide or 2-amino-N-(3,4-dimethyl-5-isoxazolyl)-4-(trifluoromethyl)benzenesulfonamide;
wherein
the terms "alkyl" and "alkoxy" refer to such groups having 1 to 10 carbon atoms, the qualifying term "lower" when present indicating a content of 1 to 4 carbon atoms;
the term "aryl" or "ar" refers to phenyl, naphthyl and biphenyl;
the terms "alkenyl" and "alkynyl" refer to such groups with 2 to 10 carbon atoms;
the term "alkylene" refers to a straight chain bridge of 1 to 5 carbon atoms connected by single bonds, which may be substituted with 1 to 3 lower alkyl groups;
the term "alkenylene" refers to a straight chain bridge of 2 to 5 carbon atoms having one or two double bonds that is connected by single bonds and may be substituted with 1 to 3 lower alkyl groups;
the term "alkynylene" refers to a straight chain bridge of 2 to 5 carbon atoms that has a triple bond therein, is connected by single bonds, and may be substituted with 1 to 3 lower alkyl groups;
the term "alkanoyl" refers to groups of the formula -C(O)alkyl;
the terms "cycloalkyl" and "cycloalkenyl" refer to cyclic hydrocarbon groups of 3 to 8 carbon atoms; and
the terms "halogen" and "halo" refer to fluorine, chlorine, bromine and iodine.

2. The compound of Claim 1, wherein R¹ is phenyl or phenoxy, optionally substituted with alkyl, alkoxy, -NZ⁷Z⁸, halo, or hydroxy.

3. The compound of Claim 2, wherein R⁷, R⁸, Z⁷ and Z⁸ are each independently hydrogen, alkyl or -C(O)alkyl.

4. The compound of Claim 3, wherein R⁷, R⁸, Z⁷ and Z⁸ are each independently hydrogen, methyl, methylethyl or acetyl.

5. The compound of Claim 1, wherein R¹ is phenyl or phenoxy, optionally substituted with alkyl, alkoxy, amino, alkylamino, dialkylamino, alkanoylamino, or hydroxy.

6. The compound of any one of Claims 1-5, wherein R² and R³ are each independently hydrogen, alkyl, or -NR⁷R⁸.

7. The compound of any one of Claims 1-5, wherein R² and R³ are each independently hydrogen, alkyl of 1 to 4 carbon atoms, amino, alkylamino, dialkylamino, or alkanoylamino.

8. The compound of any one of Claims 1-7, wherein R⁴ and R⁵ are alkyl.

9. The compound of Claim 1, which is
N-(3,4-Dimethyl-5-isoxazolyl)[1,1'-biphenyl]-2-sulfonamide;
N-(3,4-Dimethyl-5-isoxazolyl)-2-bromobenzenesulfonamide;
N-(3,4-Dimethyl-5-isoxazolyl)-2-phenoxybenzenesulfonamide;
3'-Amino-N-(3,4-dimethyl-5-isoxazolyl)[1,1'-biphenyl]-2-sulfonamide;
2-Fluoro-N-(3,4-dimethyl-5-isoxazolyl)benzenesulfonamide;
N-[3-[[(3,4-Dimethyl-5-isoxazolyl)amino]sulfonyl]-4-methylphenyl]acetamide;
5-Amino-N-(3,4-Dimethyl-5-isoxazolyl)-2-methylbenzenesulfonamide;
N-(3,4-Dimethyl-5-isoxazolyl)-2-(1-methylethyl)benzenesulfonamide;
N-(3,4-Dimethyl-5-isoxazolyl)-2-nitro-benzenesulfonamide;
2-Amino-N-(3,4-dimethyl-5-isoxazolyl)benzenesulfonamide; or
N-(3,4-Dimethyl-5-isoxazolyl)-4'-methyl[1,1'-biphenyl]-2-sulfonamide.

10. The compound of claim 1 which is
2'-Amino-N-(3,4-dimethyl-5-isoxazolyl)-[1,1'-biphenyl]-2-sulfonamide;
3'-(Dimethylamino)-N-(3,4-dimethyl-5-isoxazolyl)[1,1'-biphenyl]-2-sulfonamide;
N-(3,4-Dimethyl-5-isoxazolyl)-2-(trifluoromethyl)benzenesulfonamide;
2-Chloro-N-(3 ,4-dimethyl-5-isoxazolyl)-6-methylbenzenesulfonamide;
4'-(Dimethylamino)-N-(3,4-dimethyl-5-isoxazolyl)[1,1'-biphenyl]-2-sulfonamide;
N-[2'-[[(3,4-Dimethyl-5-isoxazolyl)amino]sulfonyl][1,1-biphenyl]-3-yl]acetamide;
N-(3,4-Dimethyl-5-isoxazolyl)-4'-propyl[1,1'-biphenyl]-2-sulfonamide;
2-(Dimethylamino)-N-(3,4-dimethyl-5-isoxazolyl)benzenesulfonamide;
2'-(Dimethylamino)-N-(3,4-dimethyl-5-isoxazolyl)-[1,1'-biphenyl]-2-sulfonamide;
N-(3,4-Dimethyl-5-isoxazolyl)-4'-(2-methylpropyl)-[1,1'-biphenyl]-2-sulfonamide;
4'-Butyl-N-(3,4-dimethyl-5-isoxazolyl)[1,1'-biphenyl]-2-sulfonamide;
N-(3,4-Dimethyl-5-isoxazolyl)-2-(1-naphthalenyl)benzenesulfonamide;
N-(3,4-Dimethyl-5-isoxazolyl)-3'-(2-methylpropyl)-[1,1'-biphenyl]-2-sulfonamide;
N-(3,4-Dimethyl-5-isoxazolyl)-4'-(2-methylpropoxy)-[1,1'-biphenyl]-2-sulfonamide;
N-(3,4-Dimethyl-5-isoxazolyl)-4'-(1-methylethoxy)-[1,1'-biphenyl]-2-sulfonamide;
N-(3,4-Dimethyl-5-isoxazolyl)-4'-(phenylmethyloxy)-[1,1'-biphenyl]-2-sulfonamide;
4'-(1,1-Dimethylethyl)-N-(3,4-dimethyl-5-isoxazolyl)-[1,1'-biphenyl]-2-sulfonamide;
N-(3,4-Dimethyl-5-isoxazolyl)-4'-methoxy-[1,1'-biphenyl]-2-sulfonamide;
N-(3,4-Dimethyl-5-isoxazolyl)-4'-[(1-methylethyl)amino][1,1'-biphenyl]-2-sulfonamide;
2-[[[2'-[[(3,4-Dimethyl-5-isoxazolyl)amino]sulfonyl]-[1,1'-biphenyl]-4-yl](1-methylethyl)amino]carbonyl]-amino]-4-methylpentanoic acid, ethyl ester ;
2'-Amino-N-(3,4-dimethyl-5-isoxazolyl)-4'-(2-methylpropyl)[1,1'-biphenyl]-2-sulfonamide;
N-[2'-[(3,4-Dimethyl-5-isoxazolyl)amino]sulfonyl][1,1'-biphenyl]-4-yl]]-N-(1-methylethyl)-β-phenylbenzenepropanamide;
2'-Nitro-N-(3,4-di methyl-5-isoxazolyl)-[1,1'-biphenyl]-2-sulfonamide ;
5-[(2-phenyl)phenyll]sulfonyl]amino]-3-methyl-4-isoxazolecarboxylic acid, ethyl ester;
N-(3-Methyl-4-phenylmethyl-5-isoxazolyl)-4'-(2-methylpropyl)-[1,1'-biphenyl]-2-sulfonamide;
N-(4,5-Dimethyl-3-isoxazolyl)-4'-(2-methylpropyl)-[1,1'-biphenyl]-2-sulfonamide;
4'-(2-Methylpropyl)-2'-Methoxy-N-(3,4-dimethyl-5-isoxazolyl)-[1,1'-biphenyl]-2-sulfonamide;
4'-(2-Methylpropyl)-2'-hydroxy-N-(3,4-dimethyl-5-isoxazolyl)-[1,1'-biphenyl]-2-sulfonamide;
N-(3-Methyl-4-nitro-5-isoxazolyl)-4'-(2-methylpropyl)-[1,1'-biphenyl]-2-sulfonamide;
N-(4-Methyl-5-isoxazolyl)-4'-(2-methylpropyl)-[1,1'-biphenyl]-2-sulfonamide;
4'-(2-Methylpropyl)-N-(4,5,6,7-tetrahydro-2,1-benzisoxazol-3-yl)-[1,1'-biphenyl]-2-sulfonamide;
4-Amino-4'-(2-methylpropyl)-N-(3,4-dimethyl-5-isoxazolyl)-[1,1'-biphenyl]-2-sulfonamide;
2'-Fluoro-4'-(2-methylpropyl)-N-(3,4-dimethyl-5-isoxazolyl)-[1,1'-biphenyl]-2-sulfonamide; or
4-Methoxy-4'-(2-methylpropyl)-N-(3,4-dimethyl-5-isoxazolyl)-[1,1'-biphenyl]-2-sulfonamide.

11. A compound of the formula 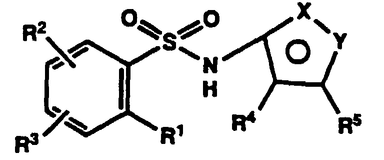 wherein:
one of X and Y is N and the other is O;
R¹, R² and R³ are each independently
(a) hydrogen, except that R¹ is other than hydrogen;
(b) alkyl, alkenyl, alkynyl, alkoxy, cycloalkyl, cycloalkylalkyl, cycloalkenyl, cycloalkenylalkyl, aryl, aryloxy, aralkyl, or aralkoxy, any of which may be substituted with Z¹, Z² and Z³;
(c) halo;
(d) hydroxyl;
(e) cyano;
(f) nitro;
(g) -C(O)H or -C(O)R⁶;
(h) -CO₂H or -CO₂R⁶;
(i) -SH, -S(O)ₙR⁶, -S(O)ₘ-OH, -S(O)ₘ-OR⁶, -O-S(O)ₘ-R⁶, -O-S(O)mOH, or -O-S(O)ₘ-OR⁶;
(j) -Z⁴-NR⁷R⁸; or
(k) -Z⁴-N(R¹¹)-Z⁵-NR⁹R¹⁰;
R⁴ and R⁵ are each independently
(a) hydrogen;
(b) alkyl, alkenyl, alkynyl, alkoxy, cycloalkyl, cycloalkylalkyl, cycloalkenyl, cycloalkenylalkyl, aryl, aryloxy, aralkyl, or aralkoxy, any of which may be substituted with Z¹, Z² and Z³;
(c) halo;
(d) hydroxyl;
(e) cyano;
(f) nitro;
(g) -C(O)H or -C(O)R⁶;
(h) -CO₂H or -CO₂R⁶;
(i) -SH, -S(O)ₙR⁶, -S(O)ₘ-OH, -S(O)ₘ-OR⁶, -O-S(O)ₘ-R⁶, -O-S(O)ₘOH, or -O-S(O)ₘ-OR⁶;
(j) -Z⁴-NR⁷R⁸;
(k) -Z⁴-N(R¹¹)-Z⁵-NR⁹R¹⁰; or
(l) R⁴ and R⁵ together are alkylene or alkenylene (either of which may be substituted with Z¹, Z² and Z³), completing a 4- to 8-membered saturated, unsaturated or aromatic ring together with the carbon atoms to which they are attached;
R⁶ is alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
R⁷ is
(a) hydrogen;
(b) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
(c) cyano;
(d) hydroxyl;
(e) -C(O)H or -C(O)R⁶;
(f) -CO₂H or -CO₂R⁶; or
(g) -SH, -S(O)ₙR⁶, -S(O)ₘ-OH, -S(O)ₘ-OR⁶, -O- S(O)ₘ₋R⁶, -O-S(O)ₘOH, or -O-S(O)ₘ-OR⁶, except when Z⁴ is -S(O)ₙ-;
R⁸ is
(a) hydrogen;
(b) -C(O)H or -C(O)R⁶, except when Z⁴ is -C(O)- and R⁷ is -C(O)H, -C(O)R⁶, -CO₂H, or -CO₂R⁶; or
(c) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³; or
R⁷ and R⁸ together are alkylene or alkenylene (either of which may be substituted with Z¹, Z² and Z³), completing a 3- to 8-membered saturated, unsaturated or aromatic ring together with the nitrogen atom to which they are attached;
R⁹ is
(a) hydrogen;
(b) hydroxyl;
(c) -C(O)H or -C(O)R⁶;
(d) -CO₂H or -CO₂R⁶;
(e) -SH, -S(O)ₙR⁶,-S(O)ₘ-OH, -S(O)ₘ-OR⁶,-O- S(O)ₘ-R⁶, -O-S(O)ₘOH, or -O-S(O)ₘ-OR⁶; or
(f) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
R¹⁰ is
(a) hydrogen;
(b) -C(O)H or -C(O)R⁶, except when Z⁵ is -C(O)- and R⁹ is -C(O)H, -C(O)R⁶, -CO₂H, or -CO₂R⁶; or
(c) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
R¹¹ is
(a) hydrogen;
(b) hydroxyl, CO₂R⁶ or CO₂H, except when one of R⁹ and R¹⁰ is hydroxyl, CO₂R⁶ or CO₂H;
(c) -C(O)H or -C(O)R⁶; or
(d) alkyl, alkenyl, alkynyl, alkoxy, cycloalkyl, cycloalkylalkyl, cycloalkenyl, cycloalkenylalkyl, aryl, or aralkyl, any of which may be substituted with Z¹, Z² and Z³;
or any two of R⁹, R¹⁰ and R¹¹ together are alkylene or alkenylene (either of which may be substituted with Z¹, Z² and Z³),
completing a 3- to 8-membered saturated, unsaturated or aromatic ring together with the atoms to which they are attached;
Z¹, Z² and Z³ are each independently
(a) hydrogen;
(b) halo;
(c) hydroxy;
(d) alkoxy;
(e) -SH, -S(O)ₙZ⁶, -S(O)m-OH, -S(O)ₘ-OZ⁶, -O- S(O)ₘ-Z⁶, -O-S(O)ₘOH, or -O-S(O)ₘ-OZ⁶;
(f) oxo;
(g) nitro;
(h) cyano;
(i) -C(O)H or -C(O)Z⁶;
(j) -CO₂H or -CO₂Z⁶;
(k) -Z⁴-NZ⁷Z⁸; or
(I) -Z⁴-NZ¹¹-Z⁵-NZ⁷Z⁸;
Z⁴ and Z⁵ are each independently
(a) a single bond;
(b) -Z⁹-S(O)ₙ-Z¹⁰-;
(c) -Z⁹-C(O)-Z¹⁰-;
(d) -Z⁹-C(S)-Z¹⁰-;
(e) -Z⁹-O-Z¹⁰-;
(f) -Z⁹-S-Z¹⁰-; or
(g) -Z⁹-O-C(O)-Z¹⁰-;
Z⁶, Z⁷ and Z⁸ are each independently hydrogen, alkyl, alkenyl, alkynyl, cycloalkyl, cycloalkylalkyl, cycloalkenyl, cycloalkenylalkyl, aryl, or aralkyl, or Z⁷ and Z⁸ together are alkylene or alkenylene, completing a 3- to 8-membered saturated, unsaturated or aromatic ring together with the nitrogen atom to which they are attached;
Z⁹ and Z¹⁰ are each independently a single bond, alkylene, alkenylene, or alkynylene;
Z¹¹ is
(a) hydrogen;
(b) hydroxyl, -CO₂H, or -CO₂Z⁶, except when one of Z⁷ and Z⁸ is hydroxyl, -CO₂H, or -CO₂Z⁶;
(c) -C(O)H or -C(O)Z⁶; or
(d) alkyl, alkenyl, alkynyl, alkoxy, cycloalkyl, cycloalkylalkyl, cycloalkenyl, cycloalkenylalkyl, aryl, or aralkyl;
or any two of Z⁷, Z⁸ and Z¹¹ together are alkylene or alkenylene, completing a 3- to 8-membered saturated, unsaturated, or aromatic ring together with the atoms to which they are attached;
m is 1 or 2; and
n is 0, 1, or 2 ;
with the proviso that such compound is not 4-amino-2-fluoro-N-(5-methyl-3-isoxazolyl)benzenesulfonamide, 2-amino-N-(5-methyl-3-isoxazolyl)-4-(trifluoromethyl )benzenesulfonamide or 2-amino-N-(3,4-dimethyl-5-isoxazolyl)-4-(trifluoromethyl)benzenesulfonamide;
wherein
the terms "alkyl" and "alkoxy" refer to such groups having 1 to 10 carbon atoms, the qualifying term "lower" when present indicating a content of 1 to 4 carbon atoms;
the term "aryl" or "ar" refers to phenyl, naphthyl and biphenyl;
the terms "alkenyl" and "alkynyl" refer to such groups with 2 to 10 carbon atoms;
the term "alkylene" refers to a straight chain bridge of 1 to 5 carbon atoms connected by single bonds, which may be substituted with 1 to 3 lower alkyl groups;
the term "alkenylene" refers to a straight chain bridge of 2 to 5 carbon atoms having one or two double bonds that is connected by single bonds and may be substituted with 1 to 3 lower alkyl groups;
the term "alkynylene" refers to a straight chain bridge of 2 to 5 carbon atoms that has a triple bond therein, is connected by single bonds, and may be substituted with 1 to 3 lower alkyl groups;
the term "alkanoyl" refers to groups of the formula -C(O)alkyl;
the terms "cycloalkyl" and "cycloalkenyl" refer to cyclic hydrocarbon groups of 3 to 8 carbon atoms; and
the terms "halogen" and "halo" refer to fluorine, chlorine, bromine and iodine.

12. The compound of any preceding claim, for use as an active pharmaceutical substance.

13. Use of the compound of any one of Claims 1-11 for the manufacture of a medicament for the treatment of an endothelin-related disorder in a mammal.

14. Use of the compound of any one of Claims 1-11 for the manufacture of a medicament for the treatment of hypertension.

15. Use of the compound of any one of Claims 1-11 for the manufacture of a medicament for the treatment of a renal, glomerular or mesangial cell disorder.

16. Use of the compound of any one of Claims 1-11 for the manufacture of a medicament for the treatment of endotoxemia.

17. Use of the compound of any one of Claims 1-11 for the manufacture of a medicament for the treatment of ischemia.

## Patentansprüche

1. Verbindung der Formel 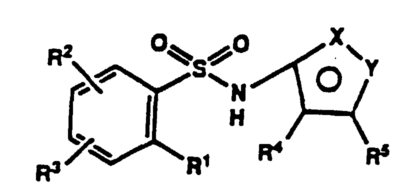 wobei
einer der Reste X und Y N ist und der andere O ist;
R¹, R² und R³ jeweils unabhängig
(a) Wasserstoffatome, ausgenommen, daß R¹ verschieden von Wasserstoff ist;
(b) Alkyl-, Alkenyl-, Alkinyl- Alkoxy-, Cycloalkyl-, Cycloalkylalkyl-, Cycloalkenyl-, Cycloalkenylalkyl-, Aryl-, Aryloxy-. Aralkyl- oder Aralkoxyreste, von denen jeder mit Z¹, Z² und Z³ substituiert sein kann;
(c) Halogenatome;
(d) Hydroxylgruppen;
(e) Cyanogruppen;
(f) Nitrogruppen;
(g) -C(O)H oder -C(O)R⁶;
(h) -CO₂H oder -CO₂R⁶;
(i) -SH, -S(O)ₙR⁶, -S(O)ₙ,-OH, -S(O)ₘ-OR⁶, -O-S(O)ₘ-R⁶, -O-S(O)ₘOH oder -O-S(O)ₘ-OR⁶;
(j) -Z⁴-NR⁷R⁸ oder
(k) -Z⁴-N(R¹¹)-Z⁵-NR⁹R¹⁰
sind;
R⁴ und R⁵ jeweils unabhängig
(a) Wasserstoffatome;
(b) Alkyl-, Alkenyl-, Alkinyl-, Alkoxy-, Cycloalkyl-, Cycloalkylalkyl-, Cycloalkenyl-, Cycloalkenylalkyl-, Aryl-, Aryloxy-, Aralkyl- oder Aralkoxyreste, von denen jeder mit Z¹, Z² und Z³ substituiert sein kann;
(c) Halogenatome;
(d) Hydroxygruppen;
(e) Cyanogruppen;
(f) Nitrogruppen;
(g) -C(O)H oder -C(O)R⁶;
(h) -CO₂H oder -CO₂R⁶;
(i) -SH, -S(O)ₙR⁶, -S(O)ₘ-OH, -S(O)ₘ-OR⁶, -O-S(O)ₘ-R⁶, -O-S(O)ₘOH oder -O-S(O)ₘ-OR⁶;
(j) -Z⁴-NR⁷R⁸ oder
(k) -Z⁴-N(R¹¹)-Z⁵-NR⁹R¹⁰
sind; oder
(l) R⁴ und R⁵ zusammen ein Alkylen- oder Alkenylenrest sind (von denen jeder mit Z¹, Z² und Z³ substituiert sein kann), und zusammen mit den Kohlenstoffatomen, an die sie gebunden sind, einen 4- bis 8-gliedrigen, gesättigten, ungesättigten oder aromatischen Ring vervollständigen;
R⁶ ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest ist, von denen jeder mit Z¹, Z² und Z³ substituiert sein kann;
R⁷
(a) ein Wasserstoffatom;
(b) ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest, von denen jeder mit Z¹, Z² und Z³ substituiert sein kann;
(c) eine Cyanogruppe;
(d) eine Hydroxylgruppe:
(e) -C(O)H oder -C(O)R⁶;
(f) -CO₂R⁶ oder
(g) -SH, -S(O)ₙR⁶, -S(O)ₘ-OH, -S(O)ₘ-OR⁶, -O-S(O)ₘ-R⁶, -O-S(O)ₘOH oder -O-S(O)ₘ-OR⁶ ist, ausgenommen wenn Z⁴ -S(O)ₙ- ist;
R⁸
(a) ein Wasserstoffatom;
(b) -C(O)H oder -C(O)R⁶, ausgenommen wenn Z⁴ -C(O)- ist und R⁷ -C(O)H, -C(O)R⁶ oder -CO₂R⁶ ist; oder
(c) ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest ist, von denen jeder mit Z¹, Z² und Z³ substituiert sein kann; oder
R⁷ und R⁸ zusammen ein Alkylen- oder Alkenylenrest sind (von denen jeder mit Z¹, Z² und Z³ substituiert sein kann), und zusammen mit dem Stickstoffatom, an das sie gebunden sind, einen 4- bis 8-gliedrigen, gesättigten, ungesättigten oder aromatischen Ring vervollständigen;
R⁹
(a) ein Wasserstoffatom;
(b) eine Hydroxylgruppe;
(c) -C(O)H oder -C(O)R⁶;
(d) -CO₂R⁶;
(e) -SH, -S(O)ₙR⁶, -S(O)ₘ-OH, -S(O)ₘ-OR⁶, -O-S(O)ₘ-R⁶, -O-S(O)ₘOH oder -O-S(O)ₘ-OR⁶; oder
(f) ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest ist, von denen jeder mit Z¹, Z² und Z³ substituiert sein kann;
R¹⁰
(a) ein Wasserstoffatom;
(b) -C(O)H oder -C(O)R⁶, ausgenommen wenn Z⁵ -C(O)- ist und R⁹ C(O)H, -C(O)R⁶ oder -CO₂R⁶ ist; oder
(c) ein Alkyl-, Alkenyl-, Alkbiyl-, Alkoxy-, Cycloalkyl-, Cycloalkylalkyl-, Cycloalkenyl-, Cycloalkenylalkyl-, Aryl- oder Aralkylrest ist, von denen jeder mit Z¹, Z² und Z³ substituiert sein kann;
R¹¹
(a) ein Wasserstoffatom;
(b) eine Hydroxylgruppe;
(c) -C(O)H, -C(O)R⁶ oder -CO₂R⁶; oder
(d) ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest ist, von denen jeder mit Z¹, Z² und Z³ substituiert sein kann;
oder irgendwelche zwei der Reste R⁹, R¹⁰ und R¹¹ zusammen ein Alkylen- oder Alkenylenrest sind (von denen jeder mit Z¹, Z² und Z³ substituiert sein kann), und zusammen mit den Atomen, an die sie gebunden sind, einen 4- bis 8-gliedrigen, gesättigten, ungesättigten oder aromatischen Ring vervollständigen;
Z¹, Z² und Z³ jeweils unabhängig
(a) Wasserstoffatome;
(b) Halogenatome;
(c) Hydroxylgruppen;
(d) Alkylreste;
(e) Alkenylreste;
(f) Aralkylreste:
(g) Alkoxyreste;
(h) Aryloxyreste;
(i) Aralkyloxyreste;
(j) -SH, -S(O)ₙZ⁶, -S(O)ₙ,-OH, -S(O)ₙ,-OZ⁶, -O-S(O)ₘ,-Z⁶, -O-S(O)ₘOH oder -O-S(O)ₘ-OZ⁶;
(k) Oxogruppen;
(l) Nitrogruppen;
(m) Cyanogruppen;
(n) -C(O)H oder -C(O)Z⁶;
(o) -CO₂H oder -CO₂Z⁶;
(p) -Z⁴-NZ⁷Z⁸;
(q) -Z⁴-N(Z¹¹)-Z⁵-Z⁶; oder
(r) -Z⁴-N(Z¹¹-Z⁵-N⁷Z⁸ sind;
Z⁴ und Z³ jeweils unabhängig
(a) Einfachbindungen;
(b) -Z⁹-S(O)ₙ-Z¹⁰;
(c) -Z⁹-C(O)-Z¹⁰;
(d) -Z⁹-C(S)-Z¹⁰;
(e) -Z⁹-O-Z¹⁰;
(f) -Z⁹-S-Z¹⁰; oder
(g) -Z⁹-O-C(O)-Z¹⁰ sind;
Z⁶, Z⁷ und Z⁸ jeweils unabhängig Wasserstoffatome, Alkyl-, Alkenyl-, Alkinyl-, Cycloalkyl-, Cycloalkylalkyl-, Cycloalkenyl-, Cycloalkenylalkyl-, Aryl- oder Aralkylreste sind oder Z⁷ und Z⁸ zusammen ein Alkylen- oder Alkenylenrest sind, und zusammen mit dem Stickstoffatom, an das sie gebunden sind, einen 3- bis 8-gliedrigen, gesättigten, ungesättigten oder aromatischen Ring vervollständigen;
Z⁹ und Z¹⁰ jeweils unabhängige Einfachbindungen, Alkylen-, Alkenylen- oder Alkinylenreste sind;
Z¹¹
(a) ein Wasserstoffatom;
(b) eine Hydroxylgruppe;
(c) -C(O)H, -C(O)Z⁶ oder -CO₂Z⁶; oder
(d) ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest ist;
oder irgendwelche zwei der Reste Z⁷, Z⁸ und Z¹¹ zusammen ein Alkylen- oder Alkenylenrest sind, und zusammen mit den Atomen, an die sie gebunden sind, einen 3- bis 8-gliedrigen, gesättigten, ungesättigten oder aromatischen Ring vervollständigen;
m 1 oder 2 ist und
n 0, 1 oder 2 ist;
mit der Maßgabe, daß diese Verbindung nicht 4-Amino-2-fluor-N-(5-methyl-3-isoxazolyl)benzolsulfonamid, 2-Amino-N-(5-methyl-3-isoxazolyl)-4-(trifluormethyl)benzensulfonamid oder 2-Amino-N-(3,4-dimethyl-5-isoxazolyl)-4-(trifluormethyl)benzolsulfonamid ist;
wobei
die Ausdrücke "Alkyl" und "Alkoxy" solche Reste betreffen, die 1 bis 10 Kohlenstoffatome haben, wobei der charakterisierende Ausdruck "Nieder-", wenn vorhanden, einen Gehalt von 1 bis 4 Kohlenstoffatomen anzeigt;
der Ausdruck "Aryl" oder "Ar" Phenyl, Naphthyl und Biphenyl betrifft;
die Ausdrücke "Alkenyl" und "Alkinyl" solche Reste mit 2 bis 10 Kohlenstoffatomen betreffen;
der Ausdruck "Alkylen" eine geradkettige, durch Einfachbindungen verbundene Brücke von 1 bis 5 Kohlenstoffatomen betrifft, die mit 1 bis 3 Niederalkylresten substituiert sein kann;
der Ausdruck "Alkenylen" eine geradkettige Brücke von 2 bis 5 Kohlenstoffatomen mit einer oder zwei Doppelbindungen betrifft, die durch Einfachbindungen verbunden ist und mit 1 bis 3 Niederalkylresten substituiert sein kann;
der Ausdruck "Alkinylen" eine geradkettige Brücke von 2 bis 5 Kohlenstoffatomen betrifft, die eine Dreifachbindung darin hat, durch Einfachbindungen verbunden ist und mit 1 bis 3 Niederalkylresten substituiert sein kann;
der Ausdruck "Alkanoyl" Reste der Formel -C(O)-Alkyl betrifft;
die Ausdrücke "Cycloalkyl" und "Cycloalkenyl" zyklische Kohlenwasserstoffreste mit 3 bis 8 Kohlenstoffatomen betreffen; und
der Ausdruck "Halogen" Fluor, Chlor, Brom und Iod betrifft.

2. Verbindung nach Anspruch 1, wobei R¹ Phenyl oder Phenoxy, gegebenenfalls substituiert mit Alkyl, Alkoxy, NZ⁷Z⁸, Halogen oder Hydroxy, ist.

3. Verbindung nach Anspruch 2, wobei R⁷, R⁸, Z⁷ und Z⁸ jeweils unabhängig Wasserstoffatome, Alkyl- oder -C(O)-Alkylreste sind.

4. Verbindung nach Anspruch 3, wobei R⁷, R⁸, Z⁷ und Z⁸ jeweils unabhängig Wasserstoffatome, Methyl-, Methylethyl- oder Acetylgruppen sind.

5. Verbindung nach Anspruch 1, wobei R¹ Phenyl oder Phenoxy, gegebenenfalls substituiert mit Alkyl, Alkoxy, Amino, Alkylamino, Dialkylamino, Alkanoylamino oder Hydroxy, ist.

6. Verbindung nach einem der Ansprüche 1-5, wobei R² und R³ jeweils unabhängig Wasserstoffatome, Alkyl oder -NR⁷R⁸ sind.

7. Verbindung nach einem der Ansprüche 1-5, wobei R² und R³ jeweils unabhängig Wasserstoffatome, Alkylreste mit 1 bis 4 Kohlenstoffatomen, Amino-, Alkylamino-, Dialkylamino- oder Alkanoylaminoreste sind.

8. Verbindung nach einem der Ansprüche 1-7, wobei R⁴ und R⁵ Alkylreste sind.

9. Verbindung nach Anspruch 1, die
N-(3,4-Dimethyl-5-isoxazolyl)-[1,1'-biphenyl]-2-sulfonamid;
N-(3,4-Dimethyl-5-isoxazolyl)-2-brombenzolsulfonamid;
N-(3,4-Dimethyl- 5-isoxazolyl)-2-phenoxybenzolsulfonamid;
3'-Amino-N-(3,4-dimethyl-5-isoxazolyl)-[1,1'-biphenyl]-2-sulfonamid;
2-Fluor-N-(3,4-dimethyl-5-isoxazolyl)benzolsulfonamid;
N- [3-[[(3,4-Dimethyl-1-isoxazolyl)amino] sulfonyl]-4-methylphenylacetamid;
5-Amino-N-(3,4-dimethyl-5-isoxazolyl)-2-methylbenzolsulfonamid;
N-(3,4-Dimethyl-5-isoxazolyl)-2-(1-methylethyl)benzolsulfonamid;
N-(3,4-Dimethyl-5-isoxazolyl)-2-nitrobenzolsulfonamid;
2-Amino-N-(3,4-dimethyl-5-isoxazolyl)benzolsuffonamid; oder
N-(3,4-Dimethyl-5-isoxazolyl)-4'-methyl[1,1'-biphenyl]-2-sulfonamid ist.

10. Die Verbindung nach Anspruch 1, die
2'-Amino-N-(3,4-dimethyl-5-isoxazolyl)-[1,1'-biphenyl]-2-sulfonamid;
3'-(Dimethylamino )-N-(3,4-dimethyl-5-isoxazolyl)-[1,1'-biphenyl]-2-sulfonamid;
N-(3,4-Dimethyl-5-isoxazolyl)-2-(trifluomethyl)benzolsulfonamid;
2-Chlor-N-(3,4-dimethyl-5-isoxazolyl)-6-methylbenzolsulfonamid;
4'-(Dimethylamino)-N-(3,4-dimethyl-5-isoxazolyl)-[1,1'-biphenyl]-2-sulfonamid;
N-[2'-[[(3,4-Dimethyl-5-isoxazolyl)amino]sulfonyl]-[1,1'-biphenyl]-3-yl]-acetamid;
N-(3,4-Dimethyl-5-isoxazolyl)-4'-propyl-[1,1'-biphenyl]-2-sulfonamid;
2-(Dimethylamino)-N-(3,4-dimethyl-5-isoxazolyl)benzolsulfonamid;
2'-(Dimethylamino)-N-(3,4-dimethyl-5-isoxazolyl)-[1,1'-biphenyl]-2-sulfonamid;
N-(3,4-Dimethyl-5-isoxazolyl)-4'-(2-methylpropyl)-[1,1'-biphenyl]-2-sulfonamid;
4'-Butyl-N-(3,4-dimethyl-5-isoxazolyl)-[1,1'-biphenyl]-2-sulfonamid;
N-(3,4-Dimethyl-5-isoxazolyl )-2-(1-naphthalenyl)-benzolsulfonamid;
N-(3,4-Dimethyl-5-isoxazolyl)-3'-(2-methylpropyl)-[1,1'-biphenyl]-2-sulfonamid;
N-(3,4-Dimethyl-5-isoxazolyl)-4'-(2-methylpropoxy)-[1,1'-biphenyl]-2-sulfonamid;
N-(3,4-Dimethyl-5-isoxazolyl)-4'-(1-methylethoxy)-[1,1'-biphenyl]-2-sulfonamid;
N-(3,4-Dimethyl-1-isoxazolyl)-4'-(phenylmethyloxy)-[1,1'-biphenyl]-2-sulfonamid;
4'-(1,1-Dimethylethyl)-N-(3,4-dimethyl-5-isoxazolyl)-[1,1'-biphenyl]-2-sulfonamid;
N-(3,4-Dimethyl-5-isoxazolyl)-4'-methoxy-[1,1'-biphenyl]-2-sulfonamid;
N-(3,4-Dimethyl-5-isoxazolyl)-4'-[(1-methylethyl)amino]-[1,1'-biphenyl]-2-sulfonamid;
2-[[[[2'-[[(3,4-Dimethyl-5-isoxazolyl)amino]sulfonyl]-[1,1'-biphenyl]-4-yl](1-methylethyl)amino]carbonyl]amino]-4-methylpentansäure, Ethylester;
2'-Amino-N-(3,4-dimethyl-5-isoxazolyl)-4'-(2-methylpropyl)-[1,1'-biphenyl]-2-sulfonamid;
N-[2'-[[(3,4-Dimethyl-5-isoxazolyl)amino]sulfonyl]-[1,1'-biphenyl]-4-yl]]-N-(1-methylethyl)-β-phenylbenzolpropanamid;
2'-Nitro-N-(3,4-dimethyl-5-isoxazolyl)-[1,1'-biphenyl]-2-suffonamid;
5-[[(2-Phenyl)phenyl]sulfonyl]amino]-3-methyl-4-isoxazolcarbonsäure, Ethylester;
N-(3-Methyl-4-phenylmethyl-5-isoxazolyl)-4'-(2-methylpropyl)-[1,1'-biphenyl]-2-sulfonamid;
N-(4,5-Dimethyl-3-isoxazolyl)-4'-(2-methylpropyl)-[1,1'-biphenyl]-2-sulfonamid;
4'-(2-Methylpropyl)-2'-methoxy-N-(3,4-dimethyl-5-isoxazolyl)-[1,1'-biphenyl]-2-sulfonamid;
4'-(2-Methylpropyl)-2'-hydroxy-N-(3,4-dimethyl-5-isoxazolyl)-[1,1'-biphenyl]-2-sulfonamid;
N-(3-Methyl-4-nitro-5-isoxazolyl)-4'-(2-methylpropyl)-[1,1'-biphenyl]-2-sulfonamid;
N-(4-Methyl-5-isoxazolyl)-4'-(2-methylpropyl)-[1,1'-biphenyl]-2-sulfonamid;
4'-(2-Methylpropyl)-N-(4,1,6,7-tetrahydro-2,1-benzisoxazol-3-yl)-[1,1'-biphenyl]-2-sulfonamid;
4-Amio-4'-(2-Methylpropyl)-N-(3,4-dimethyl-5-isoxazolyl)-[1,1'-biphenyl]-2-sulfonamid;
2'-Fluor-4'-(2-methylpropyl)-N-(3,4-dimethyl-5-isoxazolyl)-[1,1'-biphenyl]-2-sulfonamid; oder
4-Methoxy-4'-(2-methylpropyl)-N-(3,4-dimethyl-5-isoxazolyl)-[1,1'-biphenyl]-2-sulfonamid
ist.

11. Verbindung der Formel 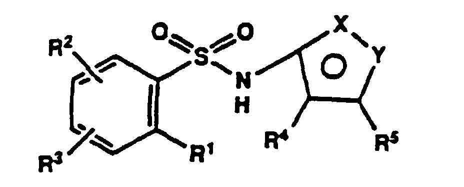 wobei
einer der Reste X und Y N ist und der andere O ist;
R¹, R² und R³ jweils unabhängig
(a) Wasserstoffatome, ausgenommen, daß R¹ verschieden von Wasserstoff ist;
(b) Alkyl-, Alkenyl-, Alkinyl-, Alkoxy-, Cycloalkyl-, Cycloalkylalkyl-, Cycloalkenyl-, Cycloalkenylalkyl-, Aryl-, Aryloxy-, Aralkyl- oder Aralkoxyreste, von denen jeder mit Z¹, Z² und Z³ substituiert sein kann;
(c) Halogenatome;
(d) Hydroxylgruppen;
(e) Cyanogruppen;
(f) Nitrogruppen;
(g) -C(O)H oder -C(O)R⁶;
(h) -CO₂H oder -CO₂R⁶;
(i) -SH, -S(O)ₙR⁶, -S(O)ₘ,-OH, -S(O)ₘ-OR⁶, -O-S(O)ₙ,-R⁶, -O-S(O)ₘOH oder -O-S(O)ₘ-OR⁶;
(j) -Z⁴-NR⁷R⁸ oder
(k) -Z⁴-N(R¹¹)-Z⁵-NR⁹R¹⁰
sind;
R⁴ und R⁵ jeweils unabhängig
(a) Wasserstoffatome;
(b) Alkyl-, Alkenyl-, Alkinyl-, Alkoxy-, Cycloalkyl-, Cycloalkylalkyl-, Cycloalkenyl-, Cycloalkenylalkyl-, Aryl-, Aryloxy-, Aralkyl- oder Aralkoxyreste, von denen jeder mit Z¹, Z² und Z³ substituiert sein kann;
(c) Halogenatome;
(d) Hydroxylgruppen;
(e) Cyanogruppen;
(f) Nitrogruppen;
(g) -C(O)H oder -C(O)R⁶;
(h) -CO₂H oder -CO₂R⁶;
(i) -SH, -S(O)ₙR⁶, -S(O)ₙ,-OH, -S(O)ₙ,-OR⁶, -O-S(O)ₘ-R⁶, -O-S(O)ₘOH oder -O-S(O)ₙ,-OR⁶;
(j) -Z⁴-NR⁷R⁸;
(k) -Z⁴-N(R¹¹)-Z⁵-NR⁹R¹⁰
sind; oder
(l) R⁴ und R⁵ zusammen ein Alkylen- oder Alkenylenrest sind (von denen jeder mit Z¹, Z² und Z³ substituiert sein kann), und zusammen mit den Kohlenstoffatomen, an die sie gebunden sind, einen 4- bis 8-gliedrigen, gesättigten, ungesättigten oder aromatischen Ring vervollständigen;
R⁶ ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest ist, von denen jeder mit Z¹, Z² und Z³ substituiert sein kann;
R⁷
(a) ein Wasserstoffatom;
(b) ein Alkyl-, Alkenyl-, Alkinyl-, Alkoxy-, Cycloalkyl-, Cycloalkylalkyl-, Cycloalkenyl-, Cydoalkenylalkyl-, Aryl- oder Aralkylrest, von denen jeder mit Z¹, Z² und Z³ substituiert sein kann;
(c) eine Cyanogruppe;
(d) eine Hydroxylgruppe;
(e) -C(O)H oder -C(O)R⁶;
(f) -CO2H oder -CO₂R⁶ oder
(g) -SFL -S(O)ₙR⁶, -S(O)ₘ-OH, -S(O)ₙ,-OR⁶, -O-S(O)ₘ-R⁶, -O-S(O)ₘOH oder -O-S(O)ₙ,-OR⁶ ist, ausgenommen wenn Z⁴ -S(O)ₙ- ist;
R⁸
(a) ein Wasserstoffatom;
(b) -C(O)H oder -C(O)R⁶, ausgenommen wenn Z⁴ -C(O)- ist und R⁷ -C(O)H, -C(O)R⁶, -CO₂H oder -CO₂R⁶ ist: oder
(c) ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest ist, von denen jeder mit Z¹, Z² und Z³ substituiert sein kann; oder
R⁷ und R⁸ zusammen ein Alkylen- oder Alkenylenrest sind (von denen jeder mit Z¹, Z² und Z³ substituiert sein kann), und zusammen mit dem Stickstoffatom, an das sie gebunden sind, einen 4- bis 8-gliedrigen, gesättigten, ungesättigten oder aromatischen Ring vervollständigen;
R⁹
(a) ein Wasserstoffatom;
(b) eine Hydroxylgruppe;
(c) -C(O)H oder -C(O)R⁶;
(d) -CO₂H oder -CO₂R⁶;
(e) -SH, -S(O)ₙR⁶, -S(O)ₘ-OH, -S(O)ₘ-OR⁶, -O-S(O)ₘ-R⁶, -O-S(O)ₘOH oder -O-S(O)ₙ,-OR⁶; oder
(f) ein Alkyl-, Alkenyl-, Alkinyl-, Alkoxy-, Cycloalkyl-, Cycloalkylalkyl-, Cycloalkenyl-, Cydoalkenylalky.1-, Aryl- oder Aralkylrest ist, von denen jeder mit Z¹, Z² und Z³ substituiert sein kann:
R¹⁰
(a) ein Wasserstoffatom:
(b) -C(O)H oder -C(O)R⁶, ausgenommen wenn Z⁵ -C(O)- ist und R⁹ -C(O)H, -C(O)R⁶, -CO₂H- oder -CO₂R⁶ ist; oder
(c) ein Alkyl-. Alkenyl-, Alkinyl-, Alkoxy-, Cycloalkyl-, Cycloalkylalkyl-, Cycloalkenyl-, Cycloalkenylalkyl-, Aryl- oder Aralkylrest ist, von denen jeder mit Z¹, Z² und Z³ substituiert sein kann;
R¹¹
(a) ein Wasserstoffatom:
(b) eine Hydroxylgruppe, -CO₂R⁶ oder -CO₂H, ausgenommen wenn einer der Reste R⁹ und R¹⁰ Hydroxyl, -CO₂R⁶ oder -CO₂H ist:
(c) -C(O)H oder -C(O)R⁶; oder
(d) ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest ist, von denen jeder mit Z¹, Z² und Z³ substituiert sein kann;
oder irgendwelche zwei der Reste R⁹, R¹⁰ und R¹¹ zusammen ein Alkylen- oder Alkenylenrest sind (von denen jeder mit Z¹, Z² und Z³ substituiert sein kann), und zusammen mit den Atomen, an die sie gebunden sind. einen 4- bis 8-gliedrigen, gesättigten, ungesättigten oder aromatischen Ring vervollständigen;
Z¹, Z² und Z³ jeweils unabhängig
(a) Wasserstoffatome;
(b) Halogenatome;
(c) Hydroxylgruppen;
(d) Alkoxyreste;
(e) -SH, -S(O)ₙZ⁶, -S(O)ₘ-OH -S(O)ₘ-OZ⁶, -O-S(O)ₘ-Z⁶, -O-S(O)ₘOH oder -O-S(O)ₘ-OZ⁶;
(f) Oxogruppen;
(g) Nitrogruppen;
(h) Cyanogruppen;
(i) -C(O)H oder -C(O)Z⁶;
(j) -CO₂H oder -CO₂Z⁶;
(k) -Z⁴-NZ⁷Z⁸; oder
(l) -Z⁴-NZ¹¹-Z⁵-NZ⁷Z⁸ sind;
Z⁴ und Z⁵ jeweils unabhängig
(a) Einfachbindungen;
(b) -Z⁹-S(O)ₙ-Z¹⁰;
(c) -Z⁹-C(O)-Z¹⁰;
(d) -Z⁹-C(S)-Z¹⁰;
(e) -Z⁹-O-Z¹⁰;
(f) -Z⁹-S-Z¹⁰; oder
(g) -Z⁹-O-C(O)-Z¹⁰ sind;
Z⁶, Z⁷ und Z⁸ jeweils unabhängig Wasserstoffatome, Alkyl-, Alkenyl-, Alkinyl-, Cycloalkyl-, Cycloalkylalkyl-, Cycloalkenyl-, Cycloalkenylalkyl-, Aryl- oder Aralkylreste sind, oder Z⁷ und Z⁸ zusammen ein Alkylen- oder Alkenylenrest sind, und zusammen mit dem Stickstoffatom, an das sie gebunden sind, einen 3- bis 8-gliedrigen, gesättigten, ungesättigten oder aromatischen Ring vervollständigen;
Z⁹ und Z¹⁰ jeweils unabhängige Einfachbindungen, Alkylen-, Alkenylen- oder Alkinylenreste sind;
Z¹¹
(a) ein Wasserstoffatom:
(b) eine Hydroxylgruppe, -CO₂H oder -CO₂R⁶, ausgenommen wenn einer der Reste Z⁷ und Z⁸ Hydroxyl, -CO₂H oder -CO₂R⁶ ist;
(c) -C(O)H oder -C(O)Z⁶; oder
(d) ein Alkyl-, Alkenyl-, Alkinyl-, Alkoxy-, Cycloalkyl-, Cycloalkylalkyl-, Cycloalkenyl-, Cycloalkenylalkyl-, Aryl- oder Aralkylrest ist;
oder irgendwelche zwei der Reste Z⁷, Z⁸ und Z¹¹ zusammen ein Alkylen- oder Alkenylenrest sind, und zusammen mit den Atomen, an die sie gebunden sind, einen 3- bis 8-gliedrigen, gesättigten. ungesättigten oder aromatischen Ring vervollständigen;
m 1 oder ist und
n 0, 1 oder 2 ist;
mit der Maßgabe, daß diese Verbindung nicht 4-Amino-2-fluor-N-(5-methyl-3-isoxazolyl)benzolsulfonamid, 2-Amino-N-(5-methyl-3-isoxazolyl)-4-(trifluormethyl)benzolsulfonamid oder 2-Amino-N-(3,4-dimethyl-5-isoxazolyl)-4-(trifluormethyl)benzolsufonamid ist;
wobei
die Ausdrücke "Alkyl" uird "Alkoxy" solche Reste betreffen, die 1 bis 10 Kohlenstoffatome haben. wobei der charakterisierende Ausdruck "Nieder-", wenn vorhanden, einen Gehalt von 1 bis 4 Kohlenstoffatomen anzeigt:
der Ausdruck "Aryl" oder "Ar" Phenyl, Naphthyl und Biphenyl betrifft;
die Ausdrücke "Alkenyl" und "Alkinyl" solche Reste mit 2 bis 10 Kohlenstoffatomen betreffen;
der Ausdruck "Alkylen" eine geradkettige, durch Einfachbindungen verbundene Brücke von 1 bis 5 Kohlenstoffatomen betrifft, die mit 1 bis 3 Niederalkylresten substituiert sein kann;
der Ausdruck "Alkenylen" eine geradkettige Brücke von 2 bis 5 Kohlenstoffatomen mit einer oder zwei Doppelbindungen betrifft, die durch Einfachbindungen verbunden ist und mit 1 bis 3 Niederalkylresten substituiert sein kann;
der Ausdruck "Alkinylen" eine geradkettige Brücke von 2 bis 5 Kohlenstoffatomen betrifft, die eine Dreifachbindung dann hat, durch Einfachbindungen verbunden ist und mit 1 bis 3 Niederalkylresten substituiert sein kann;
der Ausdruck "Alkanoyl" Reste der Formel -C(O)-Alkyl betrifft;
die Ausdrücke "Cycloalkyl" und "Cycloalkenyl" zyklische Kohlenwasserstoffreste mit 3 bis 8 Kohlenstoffatomen betreffen: und
der Ausdruck "Halogen" Fluor, Chlor, Brom und Iod betrifft.

12. Verbindung nach einem der vorstehenden Ansprüche zur Verwendung als wirksamer Arzneistoff.

13. Verwendung der Verbindung nach einem der Ansprüche 1-11 zur Herstellung eines Medikamentes zur Behandlung einer mit Endothelin in Zusammenhang stehenden Erkrankung bei einem Säuger.

14. Verwendung der Verbindung nach einem der Ansprüche 1-11 zur Herstellung eines Medikamentes zur Behandlung von Hypertonie.

15. Verwendung der Verbindung nach einem der Ansprüche 1-11 zur Herstellung eines Medikamentes zur Behandlung einer Nieren-, Gomeruli- oder Mesangiumzellerkrankung.

16. Verwendung der Verbindung nach einem der Ansprüche 1-11 zur Herstellung eines Medikamentes zur Behandlung von Endotoxämie.

17. Verwendung der Verbindung nach einem der Ansprüche 1-11 zur Herstellung eines Medikamentes zur Behandlung von Ischämie.

## Revendications

1. Composé de formule 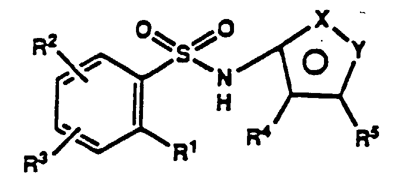 dans laquelle:
l'un de X et Y est N et l'autre est O;
R¹, R² et R³ sont chacun, indépendamment
(a) l'hydrogène, excepté que R¹ est autre que l'hydrogène;
(b) un groupe alkyle, alcényle, alcynyle, alcoxy, cycloalkyle, cycloalkylalkyle, cycloalcényle, cycloalcénylalkyle, aryle, aryloxy, aralkyle ou aralcoxy, tous ces groupes étant éventuellement substitués par Z¹, Z² et Z³;
(c) halo;
(d) hydroxyle;
(e) cyano;
(f) nitro;
(g) -C(O)H ou -C(O)R⁶;
(h) -CO₂H ou -CO₂R⁶;
(i) -SH, -S (O)ₙR⁶, -S (O)ₘ-OH, -S(O)ₘ-OR⁶, -O-S(O)ₘ-R⁶, -O-S(O)ₘOH, ou -O-S(O)ₘ-OR⁶;
(j) -Z⁴-NR⁷R⁸; ou
(k)-Z⁴-N(R¹¹)-Z⁵-NR⁹R¹⁰;
R⁴ et R⁵ sont chacun, indépendamment
(a) l'hydrogène;
(b) un groupe alkyle, alcényle, alcynyle, alcoxy, cycloalkyle, cycloalkylalkyle, cycloalcényle, cycloalcénylalkyle, aryle, aryloxy, aralkyle ou aralcoxy, tous ces groupes étant éventuellement substitués par Z¹, Z² et Z³;
(c) halo
(d) hydroxyle;
(e) cyano;
(f) nitro;
(g) -C(O)H ou -C(O)R⁶;
(h) -CO₂H ou -CO₂R⁶;
(i) -SH, -S(O)ₙR⁶, -S(O)ₘ-OH, -S (O)ₘ-OR⁶, -O-S(O)ₘ-R⁶, -O-S(O)ₘOH ou -O-S(O)ₘ-OR⁶;
(j) -Z⁴-NR⁷R⁸
(k) -Z⁴-N(R¹¹)-Z⁵-NR⁹R¹⁰; ou
(l) R⁴ et R⁵ forment ensemble un groupe alkylène ou alcénylène (tous deux éventuellement substitués par Z¹, Z² et Z³), en formant avec les atomes de carbone auxquels ils sont fixés un cycle saturé, insaturé ou aromatique de 4 à 8 chaînons;
R⁶ est un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
R⁷ est
(a) l'hydrogène;
(b) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
(c) cyano;
(d) hydroxyle;
(e) -C(O)H ou -C(O)R⁶;
(f) -CO₂R⁶; ou
(g) -SH, -S(O)ₙR⁶, -S(O)ₘ-OH, -S(O)ₘ-OR⁶, -O-S(O)ₘ-R⁶, -O-S(O)ₘOH, ou -O-S(O)ₘ-OR⁶, excepté lorsque Z⁴ est -S(O)ₙ-;
R⁸ est
(a) l'hydrogène;
(b) -C(O)H ou -C(O)R⁶, excepté lorsque Z⁴ est -C(O)- et R⁷ est -C(O)H, -C(O)R⁶, ou -CO₂R⁶; ou
(c) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³; ou
R⁷ et R⁸ forment ensemble un groupe alkylène ou alcénylène (tous deux éventuellement substitués par Z¹, Z² et Z³), en formant avec l'atome d'azote auquel ils sont fixés un cycle saturé, insaturé ou aromatique de 3 à 8 chaînons;
R⁹ est
(a) l'hydrogène;
(b) hydroxyle;
(c) -C(O)H ou -C(O)R⁶;
(d) -CO₂R⁶;
(e) -SH, -S(O)ₙR⁶, -S(O)ₘ-OH, -S(O)ₘ-OR⁶, -O-S(O)ₘ-R⁶, -O-S(O)ₘOH ou -O-S(O)ₘ₋OR⁶; ou
(f) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
R¹⁰ est
(a) l'hydrogène;
(b) -C(O)H ou -C(O)R⁶, excepté lorsque Z⁵ est -C(O)- et R⁹ est -C(O)H, -C(O)R⁶ ou -CO₂R⁶; ou
(c) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
R¹¹ est
(a) l'hydrogène;
(b) hydroxyle;
(c) -C(O)H, -C(O)R⁶ ou CO₂R⁶; ou
(d) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
ou bien deux quelconques de R⁹, R¹⁰ et R¹¹ forment ensemble un groupe alkylène ou alcénylène (tous deux éventuellement substitués par Z¹, Z² et Z³), en formant avec les atomes auxquels ils sont fixés un cycle
saturé, insaturé ou aromatique de 3 à 8 chaînons;
Z¹, Z² et Z³ sont chacun, indépendamment,
(a) l'hydrogène;
(b) halo;
(c) hydroxy;
(d) alkyle;
(e) alcényle;
(f) aralkyle;
(g) alcoxy;
(h) aryloxy;
(i) aralkyloxy;
(j) -SH, -S(O)ₙZ⁶, -S(O)ₘ-OH, -S(O)ₘ-OZ⁶, -O-S(O)ₘ-Z⁶, -O-S(O)ₘOH ou -O-S(O)ₘ-OZ⁶;
(k) oxo;
(l) nitro;
(m) cyano;
(n) -C(O)H ou -C(O)Z⁶;
(o) -CO₂H ou -CO₂Z⁶;
(p) -Z⁴-NZ⁷Z⁸;
(q) -Z⁴-N(Z¹¹)-Z⁵-Z⁶; ou
(r) -Z⁴-N(Z¹¹)-Z⁵-NZ⁷Z⁸;
Z⁴ et Z⁵ sont chacun, indépendamment,
(a) une liaison simple;
(b) -Z⁹-S(O)ₙ-Z¹⁰⁻;
(c) -Z⁹-C(O)-Z¹⁰;
(d) -Z⁹-C(S)-Z¹⁰;
(e) -Z⁹-O-Z¹⁰-;
(f) -Z⁹-S-Z¹⁰-; ou
(g) -Z⁹-O-C(O)-Z¹⁰;
Z⁶, Z⁷ et Z⁸ sont chacun, indépendamment, l'hydrogène ou un groupe alkyle, alcényle, alcynyle, cycloalkyle, cycloalkylalkyle, cycloalcényle, cycloalcénylalkyle, aryle ou aralkyle, ou bien Z⁷ et Z⁸ forment ensemble un groupe alkylène ou alcénylène, en formant avec l'atome d'azote auquel ils sont fixés un cycle saturé, insaturé ou aromatique de 3 à 8 chaînons;
Z⁹ et Z¹⁰ sont chacun, indépendamment, une liaison simple ou un groupe alkylène, alcénylène ou alcynylène;
Z¹¹ est
(a) l'hydrogène;
(b) hydroxyle;
(c) -C(O)H, -C(O)Z⁶ ou CO₂Z⁶; ou
(d) un groupe alkyle, alcényle, alcynyle, alcoxy, cycloalkyle, cycloalkylalkyle, cycloalcényle, cycloalcénylalkyle, aryle ou aralkyle;
ou bien deux quelconques de Z⁷, Z⁸ et Z¹¹ forment ensemble un groupe alkylène ou alcénylène, en formant avec les atomes auxquels ils sont fixés un cycle saturé, insaturé ou aromatique de 3 à 8 chaînons;
m est égal à 1 ou 2; et
n est égal à 0, 1 ou 2;
avec cette réserve que ce composé n'est pas le 4-amino-2-fluoro-N-(5-méthyl-3-isoxazolyl)benzènesulfonamide, le 2-amino-N-(5-méthyl-3-isoxazolyl)-4-(trifluorométhyl)benzènesulfonamide ou le 2-amino-N-(3,4-diméthyl-5-isoxazolyl)-4-(trifluorométhylbenzènesulfonamide;
les termes "alkyle" et "alcoxy" désignant de tels groupes ayant de 1 à 10 atomes de carbone, le terme qualificatif éventuel "inférieur" indiquant un contenu de 1 à 4 atomes de carbone;
le terme "aryle" ou "ar" désignant un groupe phényle, naphtyle ou biphényle;
les termes "alcényle" et "alcynyle" désignant de tels groupes avec de 2 à 10 atomes de carbone;
le terme "alkylène" désignant un pont à chaîne droite de 1 à 5 atomes de carbone raccordé par des liaisons simples, qui peut être substitué par de 1 à 3 groupes alkyle inférieur;
le terme "alcénylène" désignant un pont à chaîne droite de 2 à 5 atomes de carbone comportant une ou deux doubles liaisons, qui est raccordé par des liaisons simples et qui peut être substitué par de 1 à 3 groupes alkyle inférieur;
le terme "alcynylène" désignant un pont à chaîne droite de 2 à 5 atomes de carbone qui comporte une triple liaison, qui est raccordé par des liaisons simples, et qui peut être substitué par de 1 à 3 groupes alkyle inférieur;
le terme "alcanoyle" désignant des groupes de formule -C(O)alkyle;
les termes "cycloalkyle" et "cycloalcényle" désignant des groupes hydrocarbonés cycliques de 3 à 8 atomes de carbone; et
les termes "halogène" et "halo" désignant des atomes de fluor, de chlore, de brome ou d'iode.

2. Composé selon la fevendication 1, dans lequel R¹ est le groupe phényle ou phénoxy, facultativement substitué par alkyle, alcoxy, -NZ⁷Z⁸, halo ou hydroxy.

3. Composé selon la revendication 2, dans lequel R⁷, R⁸, Z⁷ et Z⁸ sont chacun, indépendamment, l'hydrogène ou un groupe alkyle ou -C(O)alkyle.

4. Composé selon la revendication 3, dans lequel R⁷, R⁸, Z⁷ et Z⁸ sont chacun, indépendamment, l'hydrogène ou un groupe méthyle, méthyléthyle ou acétyle.

5. Composé selon la revendication 1, dans lequel R¹ est le groupe phényle ou phénoxy, facultativement substitué par alkyle, alcoxy, amine, alkylamine, dialkylamine, alcanoylamine ou hydroxy.

6. Composé selon l'une quelconque des revendications 1 à 5, dans lequel R² et R³ sont chacun, indépendamment, l'hydrogène ou un groupe alkyle ou -NR⁷R⁸.

7. Composé selon l'une quelconque des revendications 1 à 5, dans lequel R² et R³ sont chacun, indépendamment, l'hydrogène ou un groupe alkyle de 1 à 4 atomes de carbone, amine, alkylamine, dialkylamine ou alcanoylamine.

8. Composé selon l'une quelconque des revendications 1 à 7, dans lequel R⁴ et R⁵ sont des groupes alkyle.

9. Composé selon la revendication 1, qui est
le N-(3,4-diméthyl-5-isoxazolyl) [1,1'-biphényl]-2-sulfonamide;
le N-(3,4-diméthyl-5-isoxazolyl)-2-bromobenzènesulfonamide;
le N-(3,4-diméthyl-5-isoxazolyl)-2-phénoxybenzènesulfonamide;
le 3'-amino-N-(3,4-diméthyl-5-isoxazolyl)-[1,1'-biphényl]-2-sulfonamide;
le 2-fluoro-N-(3,4-diméthyl-5-isoxazolyl)benzènesulfonamide;
le N-[3-[[(3,4-diméthyl-5-isoxazolyl)amino]sulfonyl]-4-méthylphényl]acétamide;
le 5-amino-N-(3,4-diméthyl-5-isoxazolyl)-2-méthylbenzènesulfonamide;
le N-(3,4-diméthyl-5-isoxazolyl)-2-(1-méthyléthyl)benzènesulfonamide
le N-(3,4-diméthyl-5-isoxazolyl)-2-nitrobenzènesulfonamide;
le 2-amino-N-(3,4-diméthyl-5-isoxazolyl)benzènesulfonamide; ou
le N-(3,4-diméthyl-5-isoxazolyl)-4'-méthyl[1,1'-biphényl]-2-sulfonamide.

10. Composé selon la revendication 1, qui est
le 2'-amino-N-(3,4-diméthyl-5-isoxazolyl)[1,1'-biphényl]-2-sulfonamide;
le 3'-(diméthylamino)-N-(3,4-diméthyl-5-isoxazolyl) [1,1'-biphényl]-2-sulfonamide;
le N-(3,4-diméthyl-5-isoxazolyl)-2-(trifluorométhyl)benzènesulfonamide;
le 2-chloro-N-(3,4-diméthyl-5-isoxazolyl)-6-méthylbenzènesulfonamide;
le 4'-(diméthylamino)-N-(3,4-diméthyl-5-isoxazolyl) [1,1'-biphényl]-2-sulfonamide;
le N-[2'-[[(3,4-diméthyl-5-isoxazolyl)amino]sulfonyl] [1,1'-biphényl]-3-yl]acétamide;
le N-(3,4-diméthyl-5-isoxazolyl)-4'-propyl[1,1'-biphényl]-2-sulfonamide;
le 2-(diméthylamino)-N-(3,4-diméthyl-5-isoxazolyl)benzènesulfonamide;
le 2'-(diméthylamino)-N-(3,4-diméthyl-5-isoxazolyl)-[1,1'-biphényl]-2-sulfonamide;
le N-(3,4-diméthyl-5-isoxazolyl)-4'-(2-méthylpropyl)-[1,1'-biphényl]-2-sulfonamide;
le 4'-butyl-N-(3,4-diméthyl-5-isoxazolyl) [1,1'-biphényl]-2-sulfonamide;
le N-(3,4-diméthyl-5-isoxazolyl)-2-(1-naphtalényl)benzènesulfonamide;
le N-(3,4-diméthyl-5-isoxazolyl)-3'-(2-méthylpropyl)-[1,1'-biphényl]-2-sulfonamide;
le N((3,4-diméthyl-5-isoxazolyl)-4'-(2-méthylpropoxy)-[1,1'-biphényl]-2-sulfonamide;
le N-(3,4-diméthyl-5-isoxazolyl)-4'-(1-méthyléthoxy)-[1,1'-biphényl]-2-sulfonamide;
le N-(3,4-diméthyl-5-isoxazolyl)-4'-(phénylméthoxy)-[1,1'-biphényl]-2-sulfonamide;
le 4'-(1,1-diméthyléthyl)-N-(3,4-diméthyl-5-isoxazolyl)-[1,1'-biphényl]-2-sulfonamide;
le N-(3,4-diméthyl-5-isoxazolyl)-4'-méthoxy-[1,1-biphényl]-2-sulfonamide;
le N-(3,4-diméthyl-5-isoxazolyl)-4'-[(1-méthyléthyl)amino] [1,1'-biphényl]-2-sulfonamide;
l'ester éthylique de l'acide 2-[[[[2'-[[(3,4-diméthyl-5-isoxazolyl)amino]sulfonyl]-[1,1'-biphényl]-4-yl] (1-méthyléthyl)amino]carbonyl]amino]-4-méthylpentanoïque;
le 2'-amino-N-(3,4-diméthyl-5-isoxazolyl)-4'-(2-méthylpropyl) [1,1'-biphényl]-2-sulfonamide;
le N-[2'-[[(3,4-diméthyl-5-isoxazolyl)amino]sulfonyl] [1,1'-biphényl]-4-yl]]-N-(1-méthyléthyl)-β-phénylbenzènepropanamide;
le 2'-nitro-N-(3,4-diméthyl-5-isoxazolyl)-[1,1'-biphényl]-2-sulfonamide;
l'ester éthylique de l'acide 5-[[(2-phényl)phényl]sulfonyl]amino]-3-méthyl-4-isoxazolecarboxylique;
le N-(3-méthyl-4-phénylméthyl-5-isoxazolyl)-4'-(2-méthylpropyl)-[1,1'-biphényl]-2-sulfonamide;
le N-(4,5-diméthyl-3-isoxazolyl)4'-(2-méthylpropyl)-[1,1'-biphényl]-2-sulfonamide;
le 4'-(2-méthylpropyl)-2'-méthoxy-N-(3,4-diméthyl-5-isoxazolyl)-[1,1'-biphényl]-2-sulfonamide;
le 4'-(2-méthylpropyl)-2'-hydroxy-N-(3,4-diméthyl-5-isoxazolyl)-[1,1'-biphényl]-2-sulfonamide;
le N-(3-méthyl-4-nitro-5-isoxazolyl)-4'-(2-méthylpropyl)-[1,1'-biphényl]-2-sulfonamide;
le N-(4-méthyl-5-isoxazolyl)-4'-(2-méthylpropyl)-[1,1'-biphényl]-2-sulfonamide;
le 4'-(2-méthylpropyl)-N-(4,5,6,7-tétrahydro-2,1-benzisoxazol-3-yl)-[1,1'-biphényl]-2-sulfonamide;
le 4-amino-4'-(2-méthylpropyl)-N-(3,4-diméthyl-5-isoxazolyl)-[1,1'-biphényl]-2-sulfonamide;
le 2'-fluoro-4'-(2-méthylpropyl)-N-(3,4-diméthyl-5-isoxazolyl)-[1,1'biphényl]-2-sulfonamide;
le 4-méthoxy-4'-(2-méthylpropyl)-N-(3,4-diméthyl-5-isoxazolyl)-[1,1'-biphényl]-2-sulfonamide.

11. Composé de formule 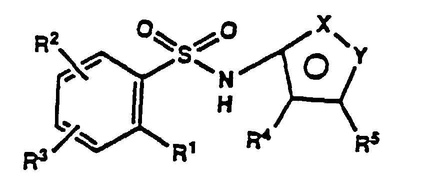 dans laquelle:
l'un de X et Y est N et l'autre est O;
R¹, R² et R³ sont chacun, indépendamment
(a) l'hydrogène, excepté que R¹ est autre que l'hydrogène;
(b) un groupe alkyle, alcényle, alcynyle, alcoxy, cycloalkyle, cycloalkylalkyle, cycloalcényle, cycloalcénylalkyle, aryle, aryloxy, aralkyle ou aralcoxy, tous ces groupes étant éventuellement substitués par Z¹, Z² et Z³;
(c) halo;
(d) hydroxyle;
(e) cyano;
(f) nitro;
(g) -C(O)H ou -C(O)R⁶;
(h) -CO₂H ou -CO₂R⁶;
(i) -SH, -S(O)ₙR⁶, -S(O)ₘ-OH, -S(O)ₘ-OR⁶, -O-S(O)ₘ-R⁶, -O-S(O)ₘOH ou -O-S(O)ₘ-OR⁶;
(j) -Z⁴-NR⁷R⁸; ou
(k) -Z⁴-N(R¹¹)-Z⁵-NR⁹R¹⁰;
R⁴ et R⁵ sont chacun, indépendamment
(a) l'hydrogène;
(b) un groupe alkyle, alcényle, alcynyle, alcoxy, cycloalkyle, cycloalkylalkyle, cycloalcényle, cycloalcénylalkyle, aryle, aryloxy, aralkyle ou aralcoxy, tous éventuellement substitués par Z¹, Z² et Z³;
(c) halo ;
(d) hydroxyle;
(e) cyano;
(f) nitro;
(g) -C(O)H ou -C(O)R⁶;
(h) -CO₂H ou -CO₂R⁶;
(i) -SH, -S(O)ₙR⁶, -S(O)ₘ-OH, -S(O)ₘ-OR⁶, -O-S(O)ₘ-R⁶, -O-S(O)ₘOH ou -O-S(O)ₘ-OR⁶;
(j) -Z⁴-NR⁷R⁸;
(k) -Z⁴-N(R¹¹)-Z⁵-NR⁹R¹⁰; ou
(l) R⁴ et R⁵ forment ensemble un groupe alkylène ou alcénylène (tous deux éventuellement substitués par Z¹, Z² et Z³), en formant avec les atomes de carbone auxquels ils sont fixés un cycle saturé, insaturé ou aromatique de 4 à 8 chaînons;
R⁶ est un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
R⁷ est
(a) l'hydrogène;
(b) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
(c) cyano;
(d) hydroxyle;
(e) -C(O)H ou -C(O)R⁶;
(f) -CO₂H ou -CO₂R⁶; ou
(g) -SH, -S(O)ₙR⁶, -S(O)ₘ-OH -S(O)ₘ-OR⁶, -O-S(O)ₘ-R⁶, -O-S(O)ₘOH ou -O-S(O)ₘ-OR⁶, excepté lorsque Z⁴ est -S(O)ₙ-;
R⁸ est
(a) l'hydrogène;
(b) -C(O)H ou -C(O)R⁶, excepté lorsque Z⁴ est -C(O)- et R⁷ est -C(O)H, -C(O)R⁶, -CO₂H ou -CO₂R⁶; ou
(c) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³; ou bien
R⁷ et R⁸ forment ensemble un groupe alkylène ou alcénylène (tous deux éventuellement substitués par Z¹, Z² et Z³), en formant avec l'atome d'azote auquel ils sont fixés un cycle saturé, insaturé ou aromatique de 3 à 8 chaînons;
R⁹ est
(a) l'hydrogène;
(b) hydroxyle;
(c) -C(O)H ou -C(O)R⁶;
(d) -CO₂H⁶ ou -CO₂R⁶;
(e) -SH, -S(O)ₙR⁶, -S(O)ₘ-OH, -S(O)ₘ-OR⁶, -O-S(O)ₘ-R⁶, -O-S(O)ₘOH ou -O-S(O)ₘ-OR⁶; ou
(f) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
R¹⁰ est
(a) l'hydrogène;
(b) -C(O)H ou -C(O)R⁶, excepté lorsque Z⁵ est -C(O)- et R⁹ est -C(O)H, -C(O)R⁶, -CO₂H ou -CO₂R⁶; ou
(c) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
R¹¹ est
(a) l'hydrogène;
(b) hydroxyle, CO₂R⁶ ou CO₂H, excepté lorsque l'un de R⁹ et R¹⁰ est un groupe hydroxyle, CO₂R⁶ ou CO₂H;
(c) -C(O)H ou C(O)R⁶; ou
(d) un groupe alkyle, alcényle, alcynyle, alcoxy, cycloalkyle, cycloalkylalkyle, cycloalcényle, cycloalcénylalkyle, aryle ou aralkyle, tous éventuellement substitués par Z¹, Z² et Z³;
ou bien deux quelconques de R⁹, R¹⁰ et R¹¹ forment ensemble un groupe alkylène ou alcénylène (tous deux éventuellement substitués par Z¹, Z² et Z³), en formant avec les atomes auxquels ils sont fixés un cycle saturé, insaturé ou aromatique de 3 à 8 chaînons;
Z¹, Z² et Z³ sont chacun, indépendamment,
(a) l'hydrogène;
(b) halo;
(c) hydroxy;
(d) alcoxy;
(e) -SH, -S(O)ₙZ⁶, -S(O)ₘ-OH, -S(O)ₘ-OZ⁶, -O-S(O)ₘ-Z⁶, -O-S(O)ₘOH ou -O-S(O)ₘ-OZ⁶;
(f) oxo;
(g) nitro;
(h) cyano;
(i) -C(O)H ou -C(O)Z⁶;
(j) -CO₂H ou -CO₂Z⁶;
(k) -Z⁴-NZ⁷Z⁸; ou
(l) -Z⁴-NZ¹¹-Z⁵-NZ⁷Z⁸;
Z⁴ et Z⁵ sont chacun, indépendamment,
(a) une liaison simple;
(b) -Z⁹-S(O)ₙ-Z¹⁰⁻;
(c) -Z⁹-C(O)-Z¹⁰;
(d) -Z⁹-C(S)-Z¹⁰⁻;
(e) -Z⁹-O-Z¹⁰-;
(f) -Z⁹-S-Z¹⁰-; ou
(g) -Z⁹-O-C(O)-Z¹⁰⁻;
Z⁶, Z⁷ et Z⁸ sont chacun, indépendamment, l'hydrogène ou un groupe alkyle, alcényle, alcynyle, cycloalkyle, cycloalkylalkyle, cycloalcényle, cycloalcénylalkyle, aryle ou aralkyle, ou bien Z⁷ et Z⁸ forment ensemble un groupe alkylène ou alcénylène, en formant avec l'atome d'azote auquel ils sont fixés un cycle saturé, insaturé ou aromatique de 3 à 8 chaînons;
Z⁹ et Z¹⁰ sont chacun, indépendamment, une liaison simple ou un groupe alkylène, alcénylène ou alcynylène;
Z¹¹ est
(a) l'hydrogène;
(b) hydroxyle; -CO₂H ou -CO₂Z⁶,excepté lorsque l'un de Z⁷ et Z⁸ est un groupe hydroxyle, ou -CO₂H ou -CO₂Z⁶;
(c) -C(O)H ou -C(O)Z⁶; ou
(d) un groupe alkyle, alcényle, alcynyle, alcoxy, cycloalkyle, cycloalkylalkyle, cycloalcényle, cycloalcénylalkyle, aryle ou aralkyle;
ou bien deux quelconques de Z⁷, Z⁸ et Z¹¹ forment ensemble un groupe alkylène ou alcénylène, en formant avec les atomes auxquels ils sont fixés un cycle saturé, insaturé ou aromatique de 3 à 8 chaînons;
m est égal à 1 ou 2; et
n est égal à 0, 1 ou 2;
avec cette réserve que ce composé n'est pas le 4-amino-2-fluoro-N-(5-méthyl-3-isoxazolyl)benzènesulfonamide, le 2-amino-N-(5-méthyl-3-isoxazolyl)-4-(trifluorométhyl)benzènesulfonamide ou le 2-amino-N-(3,4-diméthyl-5-isoxazolyl)-4-(trifluorométhyl)-benzènesulfonamide;
les termes "alkyle" et "alcoxy" désignant de tels groupes avec de 1 à 10 atomes de carbone, le terme qualificatif éventuel "inférieur" indiquant un contenu de 1 à 4 atomes de carbone;
le terme "aryle" ou "ar" désignant un groupe phényle, naphtyle ou biphényle;
les termes "alcényle" et "alcynyle" désignant de tels groupes avec de 2 à 10 atomes de carbone;
le terme "alkylène" désignant un pont à chaîne droite de 1 à 5 atomes de carbone raccordé par des liaisons simples, qui peut être substitué par de 1 à 3 groupes alkyle inférieur;
le terme "alcénylène" désignant un pont à chaîne droite de 2 à 5 atomes de carbone comportant une ou deux doubles liaisons, qui est raccordé par des liaisons simples et qui peut être substitué par de 1 à 3 groupes alkyle inférieur;
le terme "alcynylène" désignant un pont à chaîne droite de 2 à 5 atomes de carbone qui comporte une liaison triple, qui est raccordé par des liaisons simples, et qui peut être substitué par de 1 à 3 groupes alkyle inférieur;
le terme "alcanoyle" désignant des groupes de formule -C(O)alkyle;
les termes "cycloalkyle" et "cycloalcényle" désignant des groupes hydrocarbonés cycliques de 3 à 8 atomes de carbone; et
les termes "halogène" et "halo" désignant des atomes de fluor, de chlore, de brome ou d'iode.

12. Composé selon l'une quelconque des revendications précédentes, destiné à être utilisé comme substance pharmaceutique active.

13. Utilisation du composé selon l'une quelconque des revendications 1 à 11 pour la fabrication d'un médicament destiné au traitement d'un désordre lié à l'endothéline chez un mammifère.

14. Utilisation du composé selon l'une quelconque des revendications 1 à 11 pour la fabrication d'un médicament destiné au traitement de l'hypertension.

15. Utilisation du composé selon l'une quelconque des revendications 1 à 11 pour la fabrication d'un médicament destiné au traitement d'un désordre des cellules rénales, glomérulaires ou mésangiales.

16. Utilisation du composé selon l'une quelconque des revendications 1 à 11 pour la fabrication d'un médicament destiné au traitement de l'endotoxémie.

17. Utilisation du composé selon l'une quelconque des revendications 1 à 11 pour la fabrication d'un médicament destiné au traitement de l'ischémie.
